(19) Europäisches Patentamt
European Patent Office
Office européen des brevets

(11) Numéro de publication : **0 406 119 B1**

(12) **FASCICULE DE BREVET EUROPEEN**

(45) Date de publication du fascicule du brevet : **19.01.94 Bulletin 94/03**

(21) Numéro de dépôt : **90401877.7**

(22) Date de dépôt : **29.06.90**

(51) Int. Cl.$^5$ : **C07K 5/08,** C07K 1/08, C07D 403/14, C07D 453/00, C07D 209/52, A61K 31/41, A61K 31/435

# (54) Nouveaux dérivés peptidiques, leur procédé de préparation et les compositions pharmaceutiques qui les contiennent.

Le dossier contient des informations techniques présentées postérieurement au dépôt de la demande et ne figurant pas dans le présent fascicule.

(30) Priorité : **29.06.89 FR 8908672**

(43) Date de publication de la demande : **02.01.91 Bulletin 91/01**

(45) Mention de la délivrance du brevet : **19.01.94 Bulletin 94/03**

(84) Etats contractants désignés : **AT BE CH DE DK ES FR GB GR IT LI LU NL SE**

(56) Documents cités :
**FR-A- 2 460 291**
**FR-A- 2 585 709**
**BIOCHEMICAL AND BIOPHYSICAL RESEARCH COMMUNICATIONS, vol. 84, no. 4, 30 octobre 1978, pages 1097-1102, Academic Press, Inc., New York, US; C. OLIVER et al.: "Degradation of TRH and its analogues by rat serum and brain homogenate"**

(73) Titulaire : **ADIR ET COMPAGNIE**
**1 rue Carle Hébert**
**F-92415 Courbevoie Cédex (FR)**

(72) Inventeur : **Vincent, Michel**
**8 allée du Prunier Hardy**
**F-92220 Bagneux (FR)**
Inventeur : **Remond, Georges**
**9 avenue des Etats-Unis**
**F-78000 Versailles (FR)**
Inventeur : **Portevin, Bernard**
**6 rue Frédéric Passy**
**F-78990 Elancourt (FR)**
Inventeur : **Hervé, Yolande**
**16 rue Eichenberger**
**F-92800 Puteaux (FR)**
Inventeur : **Lepagnol, Jean**
**5 rue de Vlaminck**
**F-78400 Chatou (FR)**
Inventeur : **Biton, Catherine**
**121 rue de Saint-Cloud**
**F-92200 Nanterre (FR)**

EP 0 406 119 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux dérivés peptidiques, leur procédé de préparation et les compositions pharmaceutiques qui les contiennent.

Parmi les tripeptides naturels, certains tripeptides à structure cycloamidique exercent des effets centraux intéressants notamment vis-à-vis de la neurotransmission cholinergique. C'est le cas, en particulier, de la TRH (Thyrotropin-Releasing-Hormone) de structure pyroglutamyl - histidyl - prolinamide, qui est capable de s'opposer à la baisse de synthèse d'acétylcholine induite expérimentalement par narcose. Elle est également capable de potentialiser les symptômes cholinergiques centraux induits par les agonistes cholinergiques.

Cependant, la TRH est de par son métabolisme rapidement inactivée dans l'organisme. De même, elle est inactive par voie orale du fait de sa dégradation casi instantanée au niveau gastrique.

D'autres tripeptides ont été décrits (brevets FR 2.187.155, 2.287.916, 2.266.515 et 2.345.448) dans lesquels le reste pyroglutamyle est remplacé par un autre reste d'acide hétérocyclique carboxylique et qui possèdent des propriétés anti-convulsivantes et anti-dépressives. Enfin, le brevet FR 2.585.709 décrit des peptides dans lesquels le reste prolinamide est remplacé par une structure bicyclique saturée et qui sont capables de stimuler la synthèse d'AMP cyclique au niveau du tissu cérébral. Toutefois, ces dérivés n'ont pratiquement aucune activité lorsqu'administrés par voie orale.

Les composés de la présente invention, dans lesquels le reste prolinamide est remplacé par une structure bicyclique saturée nouvelle, se sont montrés très intéressants notamment par leurs propriétés à mimer et à exacerber les activités de la TRH dont ils constituent des analogues comme le démontre une étude effectuée en autoradiographie. Toutefois, le niveau d'activité des dérivés de l'invention est nettement supérieur à celui de la TRH elle-même.

En outre et de façon surprenante la nouvelle structure bicyclique saturée caractéristique des composés de l'invention rend ces dérivés actifs par voie orale contrairement aux dérivés de structure voisine précédemment connus. Cette caractéristique rend les dérivés de l'invention beaucoup plus aptes à une utilisation thérapeutique que ceux de structure proche décrits dans l'Art antérieur.

L'invention concerne plus particulièrement de nouveaux dérivés à structure cycloamidique répondant à la formule générale (I) :

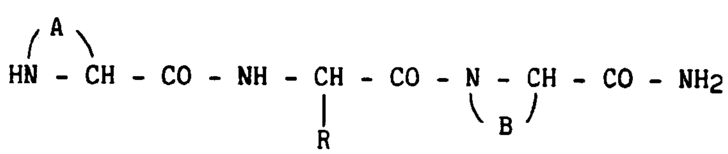

(I)

dans laquelle :

A représente avec les atomes d'azote et de carbone avec lesquels il est lié :
- un groupement oxo-2 pyrrolidinyle-5,
- un groupement oxo-2 pipéridinyle-6,
- un groupement dioxo-2,6 tétrahydro-1,2,3,6 pyrimidinyle-4,
- un groupement oxo-2 thiazolidinyle-4,
- un groupement oxo-2 azetidinyle-4 ,
- un groupement oxo-1 tétrahydro-1,2,3,4 isoquinolyle-3,

B représente avec les atomes d'azote et de carbone avec lesquels il est lié une structure polycyclique saturée choisie parmi l'aza-2 bicyclo [2.2.1] heptane ou les (dialkyl inférieurs linéaires ou ramifiés)-1,4 aza-2 bicyclo [2.2.2] octane,

R représente un atome d'hydrogène, un groupement alkyle inférieur linéaire ou ramifié, un groupement (imidazolyl-4) méthyle éventuellement substitué sur l'un des atomes d'azote par un radical alkyle inférieur, linéaire ou ramifié,

le terme inférieur indiquant que les groupements ainsi qualifiés comptent de 1 à 6 atomes de carbone, leurs énantiomères, diastéréoisomères et épimères, ainsi que leurs sels d'addition à un acide pharmaceutiquement acceptable.

Parmi les acides pharmaceutiquement acceptables, on peut citer à titre non limitatif, les acides chlorhydrique, sulfurique, tartrique, maléïque, fumarique, oxalique, méthane sulfonique, camphorique, etc...

L'invention s'étend aussi au procédé de préparation des dérivés de formule (I), caractérisé en ce que l'on protège la fonction amine d'un amino-acide de formule (II), dont on a éventuellement séparé les isomères par une technique classique de séparation :

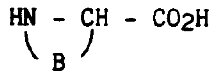

(II)

dans laquelle B a la même signification que dans la formule (I),
par un radical protecteur (P) tel que le tert.butoxycarbonyle (tBOC) ou le benzyloxycarbonyle (Z) sous l'action d'un réactif approprié pour conduire à un dérivé de formule (III) :

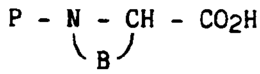

(III)

dans laquelle B et P ont la même signification que précédemment,
sur lequel on fait réagir, à une température comprise entre -15 et 0°C, en présence de triéthylamine, le chloroformiate d'éthyle puis l'ammoniaque,
pour conduire à un dérivé de formule (IV) :

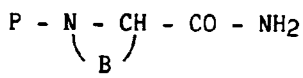

(IV)

dans laquelle B et P ont la même signification que précédemment,
que l'on déprotège par un procédé approprié comme par exemple l'action de l'acide chlorhydrique gazeux dans un solvant anhydre tel que le dioxanne ou l'acétate d'éthyle dans le cas où P = tBOC ou par hydrogénation catalytique dans le cas où P = Z,
pour conduire à un dérivé de formule (V) :

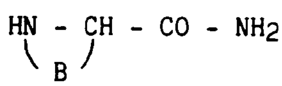

(V)

dans laquelle B a la même signification que dans la formule (I),
dont on sépare, si on le souhaite, les isomères par une technique classique de séparation,
qui est couplé avec un deuxième amino-acide protégé de formule (VI) selon la technique de couplage peptidique décrite par W. KONIG et R. GEIGER (Ber. 103, 788, 1970) :

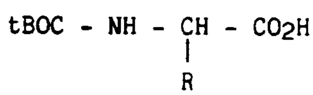

(VI)

dans laquelle R a la même signification que dans la formule (I),
pour conduire à un dérivé de formule (VII) :

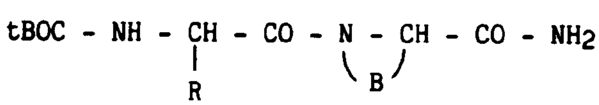

(VII)

dans laquelle R et B ont la même signification que dans la formule (I) dont on sépare, si on le souhaite, les diastéréoisomères ou énantiomères par une technique classique de séparation,
que l'on déprotège ensuite par action de l'acide chlorhydrique gazeux dans un solvant anhydre comme par exemple le dioxanne ou l'acétate d'éthyle pour conduire à un dérivé de formule (VIII) :

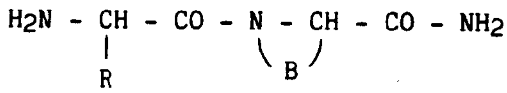

(VIII)

dans laquelle R et B ont la même signification que dans la formule (I), qui est couplé avec un troisième amino-acide, éventuellement protégé, de formule (IX), selon la technique de couplage peptidique décrite précédemment :

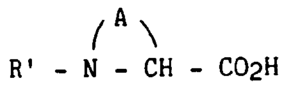

(IX)

dans laquelle R' est un hydrogène, ou un groupement protecteur tel que par exemple un benzyloxycarbonyle (Z),
ou avec un ester de cet amino-acide éventuellement protégé, pour conduire:

- ou bien à un dérivé de formule (I) dans le cas où R' est un hydrogène, qui est, si on le désire, transformé en son sel d'addition à un acide pharmaceutiquement acceptable,
- ou bien à un dérivé de formule (X) :

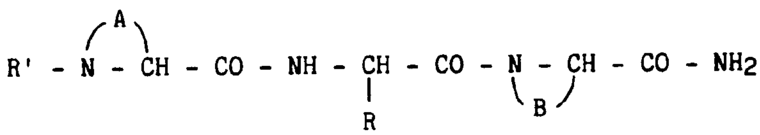

(X)

dans le cas où R' est un groupement protecteur, R et B ayant la même signification que dans la formule (I), qui est déprotégé par une technique de déprotection comme par exemple une hydrogénation catalytique pour conduire à un dérivé de formule (I), que l'on transforme, si on le désire en sel d'addition à un acide pharmaceutiquement acceptable ou dont on sépare les isomères selon une technique classique de séparation puis, si nécessaire, que l'on salifie par un acide pharmaceutiquement acceptable.

Les composés de formule (II) dans lesquels B représente avec les atomes d'azote et de carbone avec lesquels il est lié une structure (dialkyl)-1,4 aza-2 bicyclo [2.2.2] octane ainsi que les composés de formules (III), (IV), (V), (VII) et (VIII) sont nouveaux et font partie de l'invention au même titre que les composés de formule (I) dont ils constituent les intermédiaires de synthèse.

Les composés de formule (I) possèdent des propriétés pharmacologiques très intéressantes. Ces effets, s'ils sont de même nature que ceux de la TRH, s'exercent avec une intensité nettement supérieure. Ceci est confirmé par leur interaction avec les récepteurs TRHergiques observée en autoradiographie quantifiée.

D'une part, vis-à-vis du système cholinergique central, les dérivés de l'invention sont capables de restaurer les capacités neuronales de capture à haute affinité de choline lorsque cette capture qui est le facteur limitant de la synthèse d'acétylcholine, est effondrée expérimentalement par narcose barbiturique.

D'autre part, vis-à-vis du système noradrénergique central, ils sont capables de s'opposer à l'action sédative et hypotensive d'un agoniste $\alpha_2$, la clonidine, lors d'ischémie aigüe.

Ainsi, ils facilitent conjointement la neurotransmission cholinergique impliquée dans la mémoire et l'éveil, et la neurotransmission noradrénergique impliquée dans l'attention et la motivation.

Ces propriétés présentent l'avantage d'être maintenues après administration orale, ce qui rend les dérivés de l'invention beaucoup plus apte à une utilisation thérapeutique.

En particulier, cette dernière caractéristique jointe à un haut niveau d'activité rend les composés de la présente invention utilisables dans le traitement des troubles neurocomportementaux associés au vieillissement normal ou pathologique et aux maladies dégénératives aigues ou chroniques du système nerveux central comme la maladie d'Alzheimer, les désordres de conscience et de langage, schizophrénie, dépression, démence sénile, traumatisme spinal, la schlérose amiotrophique latérale, ou l'accident vasculaire cérébral.

La présente invention a également pour objet les compositions pharmaceutiques renfermant comme principe actif au moins un composé de formule générale (I) ou un de ses sels d'addition à un acide pharmacologiquement acceptable seul ou en combinaison avec un ou plusieurs excipients ou véhicules inertes, non toxiques.

Parmi les compositions pharmaceutiques selon l'invention, on pourra citer plus particulièrement celles qui

conviennent pour l'administration orale, parentérale, nasale, les comprimés simples ou dragéifiés, les comprimés sublinguaux, les sachets, les paquets, les gélules, les glossettes, les tablettes, les suppositoires, les crèmes, pommades, gels dermiques, etc...

La posologie utile varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 0,05 et 300 mg pour un traitement en 1 à 3 prises par 24 heures.

Les exemples suivants illustrent l'invention et ne la limitent en aucune façon.

Les abréviations utilisées dans les exemples sont les suivantes :

- PyroGlu à la place du radical pyrrolidone-2 carbonyle-5,
- ($N^{\tau}$-Me)His à la place de méthyl-1 histidyle dont la formule développée est la suivante :

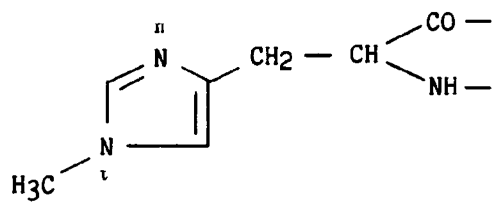

- ($N^{\pi}$-Me)His à la place de méthyl-3 histidyle dont la formule développée est la suivante :

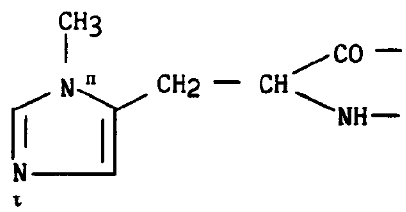

- ABH à la place de l'aza-2 carbonyl-3 bicyclo [2.2.1] heptane,
- tBOC à la place de tert-butoxycarbonyle,
- Z à la place de benzyloxycarbonyle,
- Leu à la place de leucyle,
- HomoPyroGlu à la place de pipéridinone-2 carbonyle-6,
- His à la place de Histidyle,
- OTh à la place de oxo-2 thiazolidine carbonyle-4,
- dOPyr à la place de dioxo-2,6 tétrahydro-1,2,3,6 pyrimidine carbonyle-4,
- OAz à la place de oxo-2 azétidine carbonyle-4,
- OiQ à la place de oxo-1 tétrahydro-1,2,3,4 isoquinoléine carbonyle-3,
- MIABO à la place de méthyl-4 isopropyl-1 aza-2 carbonyl-3 bicyclo [2.2.2] octane,
- dMABO à la place de diméthyl-1,4 aza-2 carbonyl-3 bicyclo [2.2.2] octane.

Les préparations indiquées ci-dessous ne permettent pas d'obtenir les produits de l'invention. En revanche, elles conduisent à l'obtention d'intermédiaires utiles dans la synthèse des produits de l'invention.

**PREPARATION A : MIABO-OH "isomères αβ"**

**STADE A : ISOPROPYL-5 METHYL-8 ETHANO-5,8 (CHLORO-4 PHENYL)-2 PENTAHYDRO-1,3,5,8,8a DIOXO-1,3 2H-IMIDAZO [1,5-a] PYRIDINE**

Dans une solution de 98 g (0,72 mole) de méthyl-1 isopropyl-4 cyclohexadiène-1,3 dans 220 ml de toluène anhydre, on ajoute 43 g (0,18mole) de pchlorophényl-3 méthoxy-5 hydantoïne (préparée selon D. BENI-, SHAI et E. GOLDSTEIN, TETRAHEDRON, 1971, 27, 3119-3127).

Ce mélange est porté à 160-170 °C en autoclave pendant 3 jours.

Le précipité formé est essoré puis recristallisé dans de l'oxyde d'isopropyle.

Rendement : 35 %

Point de fusion : 120 °C

Microanalyse élémentaire :

| | C % | H % | N % | Cl % |
|---|---|---|---|---|
| Calculé | 66,18 | 6,14 | 8,12 | 10,28 |
| Trouvé | 65,93 | 6,02 | 8,11 | 10,22 |

**STADE B : ISOPROPYL-1 METHYL-4 CARBOXY-3 AZA-2 BICYCLO [2.2.2] OCTENE -5**

6,9 g (0,02 mole) du composé préparé au stade A sont hydrolysés par chauffage à reflux dans 60 ml de soude 4N. Après acidification par HCl 6N, le composé est fixé sur une résine échangeuse d'ions puis élué par une solution ammoniacale 1N.
Le produit attendu est obtenu après concentration des solvants.
Rendement : 69 %

**STADE C : ACIDE METHYL-4 ISOPROPYL-1 AZA-2 BICYCLO [2.2.2] OCTANE CARBOXYLIQUE-3 (MIABO-OH)**

10 g (0,047 mole) du composé préparé au stade B sont hydrogénés dans 165 ml d'une solution éthanolique contenant du charbon palladié à 10 % à la pression normale.
Le produit attendu est alors obtenu sous forme de deux isomères appelés arbitrairement α, β avec un rendement de 96 %.

**PREPARATION B : dMABO-OH "isomères αβ"**

**STADE A : DIMETHYL-5,8 ETHANO-5,8 (CHLORO-4 PHENYL)-2 PENTAHYDRO-. 1,3,5,8,8a DIOXO-1,3 2H-IMIDAZO [1,5-a] PYRIDINE**

En procédant comme au stade A de la préparation A, mais en remplaçant le méthyl-1 isopropyl-4 cyclohexadiène-1,3 par le diméthyl-1,4 cyclohexadiène-1,3 (préparé selon W. BRADY et S.J. NORTON, Synthesis, 1985, 704-705), on obtient le produit attendu.
Rendement : 56 %
Point de fusion : 178 °C

Microanalyse élémentaire :

| | C % | H % | N % | Cl % |
|---|---|---|---|---|
| Calculé | 64,46 | 5,41 | 8,84 | 11,19 |
| Trouvé | 64,56 | 5,42 | 8,64 | 10,99 |

**STADE B : DIMETHYL-1,4 CARBOXY-3 AZA-2 BICYCLO [2.2.2] OCTENE-5**

En procédant comme au stade B de la préparation A, on obtient le produit attendu.
Rendement : 60 %

Microanalyse élémentaire :

| | C % | H % | N % |
|---|---|---|---|
| Calculé | 66,27 | 8,34 | 7,73 |
| Trouvé | 66,22 | 7,98 | 7,89 |

**STADE C : ACIDE DIMETHYL-1,4 AZA-2 BICYCLO [2.2.2] OCTANE CARBOXYLIQUE-3 (dMABO - OH)**

En procédant comme au stade C de la préparation A, on obtient le produit attendu sous forme de deux isomères arbitrairement appelés α,β avec un rendement de 80 %.

**EXEMPLE 1 : (S)PyroGlu-(S)($N^{\tau}$-Me)His-(1S,3S,4R)ABH-$NH_2$**

**STADE A : tBOC-ABH-OH (mélange de quatre isomères)**

La matière première utilisée est l'aza-2 carboxy-3 bicyclo [2.2.1] heptane, en abrégé ABH-OH, obtenu selon le mode opératoire décrit dans le brevet Fr 2525604. Ce mode opératoire permet l'obtention simultanée des quatre isomères pour lesquels la jonction de cycle est cis.

Dans un mélange contenant 70 ml de dioxanne et 30 ml d'eau, dissoudre 0,039 mole d'ABH-OH (mélange de quatre isomères), refroidir à 0°C, ajouter 39 ml de soude N puis goutte à goutte une solution de 0,039 mole de dicarbonate de ditertiobutyle dans 100 ml de dioxanne.

Laisser sous agitation 30 minutes à une température comprise entre 0 et 5°C puis 2 heures à température ambiante. Evaporer les solvants sous pression réduite.

Reprendre le résidu par de l'eau, acidifier par de l'acide citrique jusqu'à pH=4 et extraire la phase aqueuse avec de l'acétate d'éthyle. Laver la phase organique par une solution aqueuse de chlorure de sodium à 10 %, sécher sur sulfate de calcium anhydre, filtrer et concentrer sous pression réduite.

Recristalliser dans le n-pentane.

Rendement : 71 %

Infrarouge (nujol) : νCO (acide) : 1740 $cm^{-1}$
ν CO (carbamate) : 1640 $cm^{-1}$

Microanalyse élémentaire :

| | C % | H % | N % |
|---|---|---|---|
| calculé : | 59,73 | 7,94 | 5,80 |
| trouvé : | 59,56 | 7,72 | 5,80 |

**STADE B : tBOC-ABH-$NH_2$(mélange de quatre isomères)**

Dans une solution de 6,7 g (0,028 mole) de tBOC-ABH-OH obtenus au stade A dans 80 ml de tétrahydrofuranne refroidie dans un mélange glace/sel (température:-10°C), on ajoute 4,27 ml de triéthylamine puis 2,97 ml de chloroformiate d'éthyle fraîchement distillé en solution dans 20 ml de tétrahydrofuranne. Il se forme un précipité que l'on laisse sous agitation pendant un quart d'heure à -10 °C. 5,27 ml de solution d'ammoniaque concentrée sont alors ajoutés et l'ensemble est laissé sous agitation 30 minutes à -10 °C. On laisse revenir à température ambiante. Après évaporation des solvants sous pression réduite, le résidu est repris par une solution aqueuse d'acide citrique à pH=4.

La phase aqueuse acide est extraite par de l'acétate d'éthyle. La phase organique est alors lavée par une solution aqueuse de bicarbonate de soude, puis par de l'eau et enfin séchée sur sulfate de calcium anhydre. Filtrer et concentrer sous pression réduite.

Rendement : 90 %

Résonance magnétique nucléaire du proton ($CDCl_3$) :

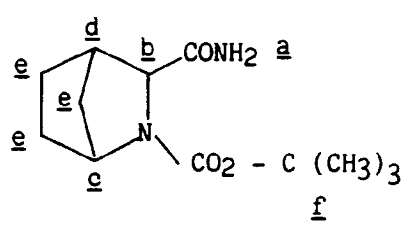

a δ entre 6,2 et 5,7 ppm (2H,m)
b δ = 4,5 ppm (1H,m)
c δ = 4,1 ppm (1H,m)
d δ = 2,9 ppm (1H,m)

e δ = 1,6 ppm (6H,m)
f δ = 1,4 ppm (9H,s)

**STADE C : ABH-$NH_2$, HCl (mélange de quatre isomères)**

Saturer de gaz chlorhydrique une solution de 87,5 g (0,365 mole) de tBOC-ABH-$NH_2$ dans 1 litre de dioxanne anhydre et laisser sous agitation 18 heures à température ambiante.
Evaporer le dioxanne et reprendre le résidu par 300 ml d'éther anhydre. Essorer, laver et sécher le produit obtenu.

Rendement : 86 %
Résonance magnétique nucléaire du proton ($CDCl_3$) :

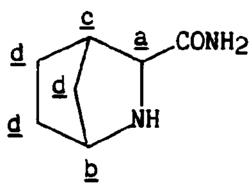

, HCl

a δ= 4,4 ppm (1H,m)
b δ = 4,1 ppm (1H,m)
c δ = 3,0 ppm (1H,m)
d δ entre 2 et 1,3 ppm (6H,m)

**STADE D : tBOC (S)($N^{\tau}$-Me)His-ABH-NH2 (mélange d'isomères)**

En utilisant la méthode de couplage peptidique (DCC/HOBT) décrite par W. KONIG et R. GEIGER (Ber, 103, 788, 1970) et le diméthylformamide comme solvant, on prépare à partir de 0,0181 mole de ABH-$NH_2$ obtenu au stade précédent et de 0,0181 mole de tBOC(S)($N^{\tau}$-Me)His-OH, le tBOC(S)($N^{\tau}$-Me)His-ABH-$NH_2$.

Rendement : 85 %

Le mélange de diastéréoisomères obtenu est séparé par chromatographie sur colonne de silice en éluant par un mélange dichlorométhane/méthanol/ammoniaque dans les proportions 9/1/0,1. La condensation de Diels-Alder utilisée pour l'obtention d'ABH-OH (brevet FR 2525604) implique qu'il est obtenu avec des jonctions de cycle cis, c'est-à-dire sous forme de deux couples d'énantiomères. Le couplage avec la tBOC(S)($N^{\tau}$-Me)His-OH donne donc lieu à 4 diastéréoisomères qui sont séparés dans les conditions décrites précédemment et dont les configurations absolues ont été déterminées par rayons X.

**STADE E : (S)($N^{\tau}$-Me)His-(1S,3S,4R) ABH-$NH_2$, dichlorhydrate**

L'isomère tBoc-(S)($N^{\tau}$-Me)His-(1S,3S,4R)ABH-$NH_2$ est déprotégé selon la méthode décrite au stade C.

Rendement : 95 %

Ce composé peut également être obtenu en remplaçant au stade D l'ABH-$NH_2$ (mélange d'isomères) par l'isomère (1S,3S,4R)ABH-$NH_2$ obtenu au stade E de l'exemple 5, (dans ce cas, la séparation des diastéréoisomères décrite au stade précédent est inutile).

**STADE F : Z (S)PyroGlu-(S)($N^{\tau}$-Me)His-(1S,3S,4R)ABH-$NH_2$**

En utilisant la méthode de couplage peptidique décrite par G.W. Anderson et J.E. Zimmerman (JACS,85,3039, 1963), on fait réagir 0,033 mole de (S)($N^{\tau}$-Me)His-(1S,3S,4R)ABH-$NH_2$ obtenu au stade précédent avec 0,033mole de l'ester activé de N-hydroxysuccinimide de Z-(S)PyroGlu-OH dans 100 ml de diméthylformamide et 0,066 mole de triéthylamine.

On obtient, après traitement usuel et chromatographie sur colonne de silice en utilisant comme éluant le mélange acétone/eau dans les proportions 95/5, le produit attendu.

Rendement : 57 %

**STADE G : (S)PyroGlu-(S)($N^{\tau}$-Me)His-(1S,3S,4R)ABH-$NH_2$**

Le produit obtenu au stade précédent est déprotégé par hydrogénation catalytique dans l'éthanol en présence de palladium sur charbon utilisé comme catalyseur.
Après filtration du catalyseur, évaporation du solvant, le produit est chromatographié sur silice en utilisant comme éluant un mélange dichlorométhane/méthanol/ammoniaque dans les proportions 80/20/1.

Microanalyse élémentaire :

| | | C % | H % | N % |
|---|---|---|---|---|
| calculé | : | 56,70 | 6,51 | 20,88 |
| trouvé | : | 56,35 | 6,08 | 20,80 |

Résonance magnétique nucléaire du proton ($D_2O$) :

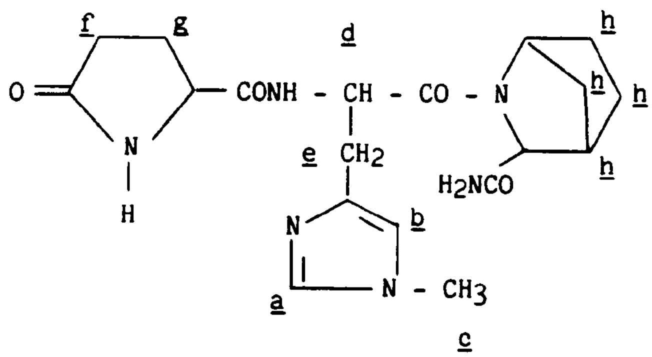

a δ = 7,6 ppm (1H,s)
b δ = 7,0 ppm (1H,s)
c δ = 3,7 ppm (3H,s)
d δ = 5 ppm (1H,t)
e δ = 3,1 ppm (2H,d)
f+g δ entre 2,3 et 2,9 ppm (4H,s)
h δ entre 1,1 et 2,1 ppm (7H,m)

**EXEMPLE 2 : (S)PyroGlu-(S)($N^{\tau}$-Me)His-(1R,3S,4S)ABH-$NH_2$**

En procédant comme dans l'exemple 1, mais en remplaçant au stade E l'isomère tBoc-(S)($N^{\tau}$-Me)His-(1S,3S,4R)ABH-$NH_2$ par l'isomère tBOC-(S)($N^{\tau}$-Me)His-(1R,3S,4S)ABH-$NH_2$ obtenu au stade D, on obtient le produit attendu.

Résonance magnétique nucléaire du proton ($D_2O$) :

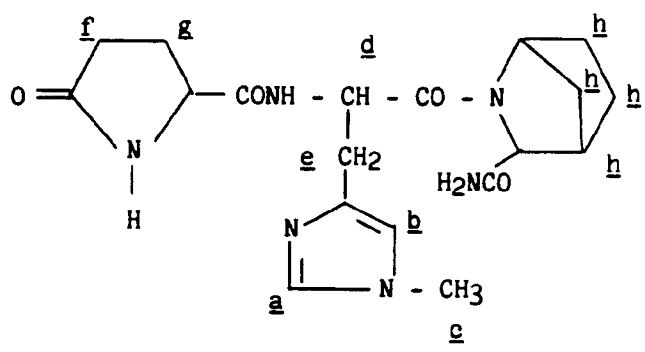

a δ = 8,0 ppm (1H,s)

b δ = 7,3 ppm (1H,s)
c δ = 3,9 ppm (3H,s)
e δ = 3,3 ppm (2H,d)
d δ = entre 4,2 et 5,3 ppm (1H,m)
f+g δ entre 2,5 et 3,7 ppm (4H,m)
h δ entre 1,4 et 2,3 ppm (7H,m)

**EXEMPLE 3 : (S)PyroGlu-(S)(N$^{\tau}$-Me)His-(1S,3R,4R)ABH-$NH_2$**

En procédant comme dans l'exemple 1, mais en remplaçant au stade E l'isomère tBoc-(S)(N$^{\tau}$-Me)His-(1S,3S,4R)ABH-$NH_2$ par l'isomère tBOC-(S)(N$^{\tau}$-Me)His-(1S,3R,4R)ABH-$NH_2$ obtenu au stade D, on obtient le produit attendu.

Résonance magnétique nucléaire du proton ($D_2O$) :

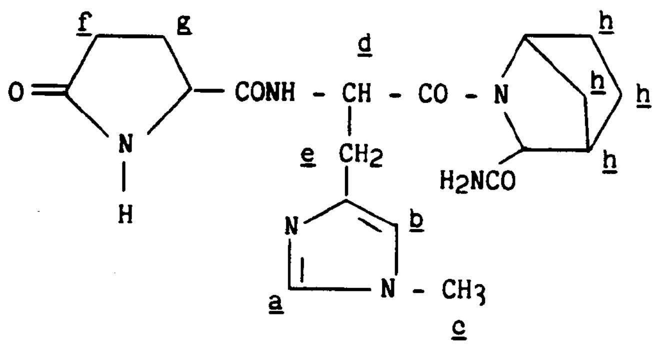

a δ = 7,6 ppm (1H,s)
b δ = 6,0 ppm (1H,s)
c δ = 3,7 ppm (3H,s)
d δ = 4,9 ppm (1H,m)
e δ = 3,0 ppm (2H,m)
f+g δ entre 2,3 et 2,8 ppm (4H,m)
h δ entre 1,3 et 2,1 ppm (7H,m)

**EXEMPLE 4 : (S)PyroGlu-(S)(N$^{\tau}$-Me)His-(1R,3R,4S)ABH-$NH_2$**

En procédant comme dans l'exemple 1, mais en remplaçant au stade E l'isomère tBoc-(S)(N$^{\tau}$-Me)His-(1S,3S,4R)ABH-$NH_2$ par l'isomère tBoc-(S)(N$^{\tau}$-Me)His-(1R,3R,4S)ABH-$NH_2$ obtenu au stade D, on obtient le produit attendu.

Résonance magnétique nucléaire du proton ($D_2O$) :

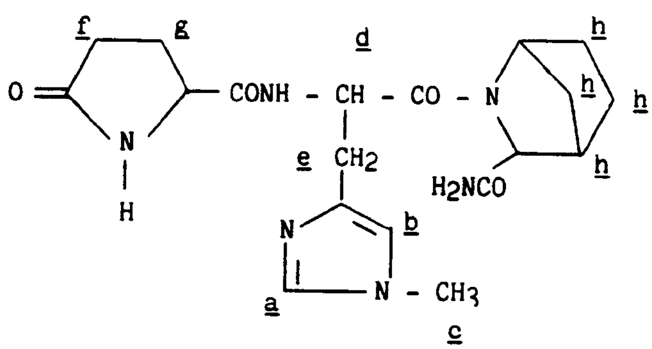

a δ = 7,7 ppm (1H,s)
b δ = 7,0 ppm (1H,s)
c δ = 3,7 ppm (3H,s)

e δ = 3,0 ppm (2H,d)
d δ entre 4,1 et 5 ppm (1H,m)
f+g δ entre 2,3 et 2,9 ppm (4H,m)
h δ entre 1,5 et 2,2 ppm (7H,m)

**EXEMPLE 5 : (S)PyroGlu-(S)Leu-(1S,3S,4R)ABH-$NH_2$**

**STADE A : ABH-OH [mélange d'isomères (1S,3S,4R)/(1R,3R,4S)]**

100 g du mélange racémique des quatre isomères d'ABH-OH obtenu selon la méthode décrite dans le brevet Fr. 2525604 sont dissous à chaud dans 1400 $cm^3$ de méthanol anhydre. On laisse cette solution refroidir lentement et sous agitation pendant 20 heures. Le précipité formé est alors filtré et rincé par 50 $cm^3$ de méthanol anhydre puis séché ; 31,5 g d'ABH-OH (mélange d'isomères (1S,3S,4R)/1R,3R,4S)) sont ainsi obtenus. Le filtrat est alors amené à sec ; le résidu obtenu est recristallisé dans de l'éthanol anhydre puis dans du méthanol anhydre et conduit à 15 g supplémentaires de produit attendu.
La pureté de ce mélange d'isomères est suivie par chromatographie liquide dans les conditions suivantes :

| | | |
|---|---|---|
| Colonne : | longueur : 25 cm | |
| | diamètre intérieur : 4,5 mm | |
| Phase stationnaire : | Ultra Base (taille des particules : 5 μm) | |
| Phase mobile : | phase aqueuse contenant | |
| | 1,25 % d'acide trifluoroacétique | : 200 volumes |
| | acétonitrile : 5 volumes | |
| Débit : | 1 ml/min | |
| Température : | 20 °C | |

Temps de rétention :
ABH - OH [mélange d'isomères (1S,3S,4R)/(1R,3R,4S)]* : 5,5 min
ABH - OH [mélange d'isomères (1R,3S,4S)/(1S,3R,4R)]* : 6,1 min

De même, si on utilise, le mélange de 2 isomères ayant un temps de rétention de 6,1 minutes comme matière première dans le stade A de l'exemple 1, on obtient au stade D de l'exemple 1, les isomères (1R,3S,4S) et (1S,3R,4R) du tBoc-(S)($N^\tau$-Me)His-ABH-$NH_2$.

**STADE B : tBOC-ABH-OH [mélange d'isomères (1S,3S,4R)/(1R,3R,4S)]**

En procédant comme au stade A de l'exemple 1, on obtient le produit attendu.
Rendement : 91 %

| | |
|---|---|
| Infrarouge (nujol) : | νCO (acide) : 1740 $cm^{-1}$ |
| | ν CO (carbamate) : 1640 $cm^{-1}$ |

**STADE C : tBOC-ABH-$NH_2$[mélange d'isomères (1S,3S,4R)/(1R,3R,4S)]**

En procédant comme au stade B de l'exemple 1, on obtient le produit attendu.
Rendement : 97 %
Résonance magnétique nucléaire du proton ($CDCl_3$) :

∗ Définition : Le premier mélange d'isomères (temps de rétention 5,5 minutes) ainsi obtenu, lorsqu' il est utilisé comme matière première dans le stade A de l'exemple 1, (au lieu du mélange des quatre isomères obtenus selon le brevet Fr 2525604) permet, en procédant selon les protocoles décrits aux stades A, B, C, D de l'exemple 1, d'obtenir les deux seuls isomères (1S,3S,4R) et (1R,3R,4S) du tBoc-(S)($N^\tau$-Me)His ABH-$NH_2$.

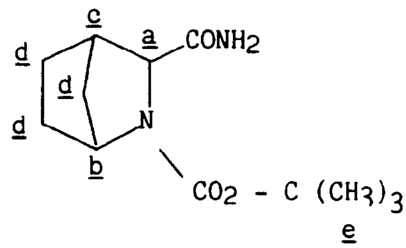

a δ = 4,5 ppm (1H,m)
b δ = 4,1 ppm (1H,m)
c δ = 2,9 ppm (1H,m)
d δ = 1,6 ppm (6H,m)
e δ = 1,4 ppm (9H,s)

**STADE D : ABH-$NH_2$, HCl [mélange d'isomères (1S,3S,4R)/(1R,3R,4S)]**

En procédant comme au stade C de l'exemple 1, on obtient le produit attendu.
Rendement : 86 %

Microanalyse élémentaire :

| | C % | H % | N % | Cl % |
|---|---|---|---|---|
| Calculé | 47,60 | 7,42 | 15,86 | 20,07 |
| Trouvé | 47,90 | 7,40 | 15,83 | 19,82 |

**STADE E : (1S,3S,4R)ABH-$NH_2$**

25,5 g (0,155 mole) d'ABH - $NH_2$, HCl [mélange d'isomères (1S,3S,4R)/(1R,3R,4S)] obtenus au stade précédent sont dissous dans 250 $cm^3$ d'eau. Cette solution est neutralisée par de la soude 10 N et amenée à sec. Le résidu agité dans 200 $cm^3$ d'isopropanol anhydre est filtré. Le filtrat est alors évaporé, repris par 250 $cm^3$ de chlorure de méthylène, filtré pour éliminer les traces de chlorure de sodium puis évaporé à sec. Après dissolution dans 400 $cm^3$ de méthanol anhydre, 20,8 g (0,139 mole) d'acide D(-) tartique sont ajoutés à cette solution puis l'ensemble est porté à reflux sous agitation jusqu'à dissolution complète. L'ensemble est refroidi lentement et sous agitation pendant 18 heures. Le précipité formé est filtré et rincé par 25 $cm^3$ de méthanol. Cette opération de purification sera renouvelée jusqu'à obtention d'un isomère optiquement pur. La pureté énantiomérique est suivie par chromatographie liquide dans les conditions suivantes après dérivation par le réactif de MOSHER.

Colonne : longueur : 15 cm
diamètre intérieur : 6,0 mm
Phase stationnaire : ASAHI PAK ODP-50 (taille des particules : 5 µm)
Phase mobile : acétonitrile : 65 volumes
eau : 35 volumes
$H_2SO_4$ : 0,5 volumes
Débit : 1 ml/min
Temps de rétention :
(1S,3S,4R)ABH-$NH_2$ : 6,4 min
(1R,3R,4S)ABH-$NH_2$ : 6,8 min

Le tartrate d'(1S,3S,4R)ABH-$NH_2$ est alors dissous dans l'eau, fixé sur une résine échangeuse d'ions puis élué par une solution ammoniacale à 10 %.
Après évaporation, on obtient le produit attendu.
Rendement : 60 %

**STADE F : tBOC-(S)Leu-(1S,3S,4R)ABH-$NH_2$**

En procédant comme au stade D de l'exemple 1, mais en remplaçant la tBOC-(S)(Nτ-Me)His-OH par la tBOC-(S)Leu-OH, on obtient le produit attendu.

**STADE G : (S)Leu-(1S,3S,4R)ABH-$NH_2$, chlorhydrate**

En procédant comme au stade E de l'exemple 1, on obtient le produit attendu.
Rendement : 87 %

**STADE H : Z(S)PyroGlu-(S)Leu-(1S,3S,4R)ABH-$NH_2$**

En procédant comme au stade F de l'exemple 1, mais en remplaçant le (S)($N^{\tau}$-Me)His-(1S,3S,4R)ABH-$NH_2$ par le (S)Leu-(1S,3S,4R)ABH-$NH_2$ obtenu au stade précédent, on obtient le produit attendu.
Rendement : 72 %

**STADE I : (S)PyroGlu-(S)Leu-(1S,3S,4R)ABH-$NH_2$**

En procédant comme au stade G de l'exemple 1, mais en remplaçant le Z(S)-PyroGlu(S)($N^{\tau}$-Me)His-(1S,3S,4R)ABH-$NH_2$ par le Z(S)PyroGlu-(S)Leu-(1S,3S,4R)ABH-$NH_2$ obtenu au stade précédent, on obtient le produit attendu.
Rendement : 57 %

Microanalyse élémentaire :

| | C % | H % | N % |
|---|---|---|---|
| Calculé | 59,32 | 7,74 | 15,37 |
| Trouvé | 59,11 | 7,93 | 15,66 |

**EXEMPLE 6 : (S)PyroGlu-(S)($N^{\pi}$-Me)His-(1S,3S,4R)ABH-$NH_2$**

Les stades A à E sont identiques aux stades A à E de l'exemple 5.

**STADE F : tBOC(S)($N^{\pi}$-Me)His-(1S,3S,4R)ABH-$NH_2$**

En procédant comme au stade F de l'exemple 5, mais en remplaçant la tBOC-(S)Leu-OH par la tBOC-(S)($N^{\pi}$-Me)His-OH, on obtient le produit attendu.
Rendement : 83 %

**STAGE G : (S)($N^{\pi}$-Me)His-(1S,3S,4R)ABH-$NH_2$, dichlorhydrate**

En procédant comme au stade G de l'exemple 5, mais en remplaçant le tBOC-(S)Leu-(1S,3S,4R)ABH-$NH_2$ par le tBOC-(S)($N^{\pi}$-Me)His-(1S,3S,4R)ABH-$NH_2$ obtenu au stade précédent, on obtient le produit attendu.
Rendement : 81 %

**STADE H : Z (S)PyroGlu-(S)($N^{\pi}$-Me)His-(1S,3S,4R)ABH-$NH_2$**

En procédant comme au stade H de l'exemple 5, mais en remplaçant le (S)Leu-(1S,3S,4R)ABH-$NH_2$ par le (S)($N^{\pi}$-Me)His-(1S,3S,4R)ABH-$NH_2$, dichlorhydrate obtenu au stade précédent, on obtient le produit attendu.

**STADE I : (S) PyroGlu-(S)($N^{\pi}$-Me)HiS-(1S,3S,4R)ABH-$NH_2$**

En procédant comme au stade I de l'exemple 5, mais en remplaçant le Z(S)PyroGlu-(S)Leu-(1S,3S,4R)ABH-$NH_2$ par le Z(S)PyroGlu-(S)($N^{\pi}$-Me)His-(1S,3S,4R)ABH-$NH_2$ obtenu au stade précédent, on obtient le produit attendu.
Rendement : 66 %

Résonance magnétique nucléaire du proton ($D_20$) :

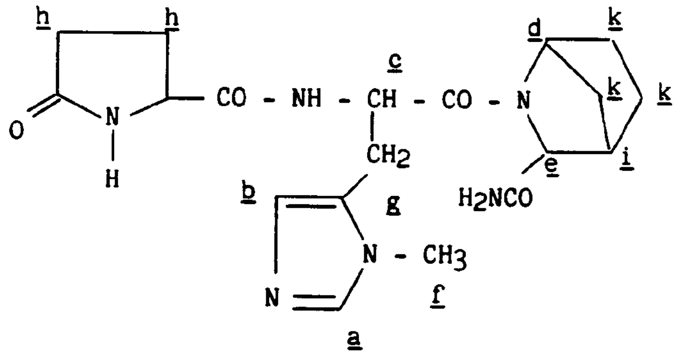

a $\delta$ = 7,5 ppm (1H,s)
b $\delta$ = 6,8 ppm (1H,s)
c $\delta$ = 5,0 ppm (1H,m)
d $\delta$ = 4,5 ppm (1H,m)
e $\delta$ = 4,3 ppm (1H,m)
f $\delta$ = 3,6 ppm (3H,s)
g $\delta$ = 3,0 ppm (2H,m)
h $\delta$ = 2,5 ppm (4H,m)
i $\delta$ = 2,7 ppm (1H,m)
k $\delta$ = 1,6 ppm (6H,m)

**EXEMPLE 7 : (S)HomoPyroGlu-(S)(N$^{\pi}$-Me)His-(1S,3S,4R)ABH-$NH_2$**

Les stades A à G sont identiques aux stades A à G de l'exemple 6.

**STADE H : (S)HomoPyroGlu-(S)(N$^{\pi}$-Me)His-(1S,3S,4R)ABH-$NH_2$**

En procédant comme au stade H de l'exemple 6, mais en remplaçant l'ester activé de N-hydroxysuccinimide de Z(S)PyroGlu-OH par l'ester activé d'hydroxysuccinimide de (S)HomoPyroGlu-OH, on obtient le produit attendu.

Rendement : 83 %

Microanalyse élémentaire :

| | C % | H % | N % |
|---|---|---|---|
| Calculé | 57,68 | 6,78 | 20,18 |
| Trouvé | 57,60 | 6,75 | 19,88 |

**EXEMPLE 8 : (S)HomoPyroGlu-(S)Leu-(1S,3S,4R)ABH-$NH_2$**

Les stades A à G sont identiques aux stades A à G de l'exemple 5.

**STADE H : (S)HomoPyroGlu-(S)Leu-(1S,3S,4R)ABH-$NH_2$**

En procédant comme au stade H de l'exemple 5 mais en remplaçant l'ester activé d'hydroxysuccinimide de Z (S)PyroGlu-OH par l'ester activé de N-hydroxysuccinimide de (S)HomoPyroGlu -OH, on obtient le produit attendu.

Rendement : 64 %

Résonance magnétique nucléaire du proton (DMSO $d_6$) :

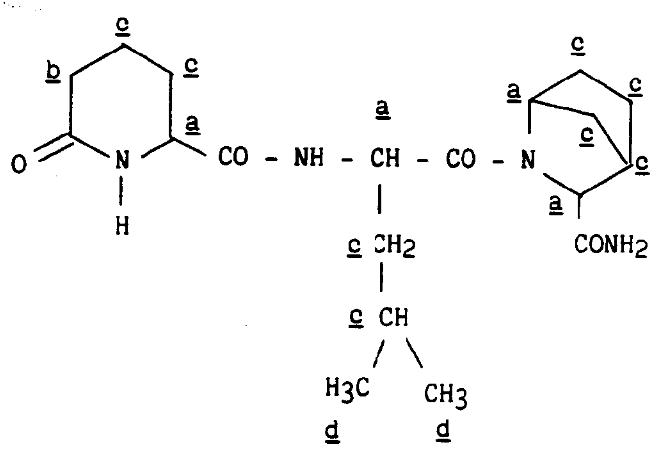

a δ entre 4 et 4,6 ppm (4H,m)
b δ = 2,6 ppm (2H,m)
c δ entre 2,2 et 1,4 ppm (14H,m)
d δ = 0,9 ppm (6H,d)

**EXEMPLE 9 : (S)PyroGlu-(S)His-(1S,3S,4R)ABH-NH$_2$**

En procédant comme dans l'exemple 5, mais en remplaçant au stade F la tBOC-(S)Leu-OH par la tBOC(S)His-OH, on obtient le produit attendu.

Résonance magnétique nucléaire du proton (DMSO $d_6$) :

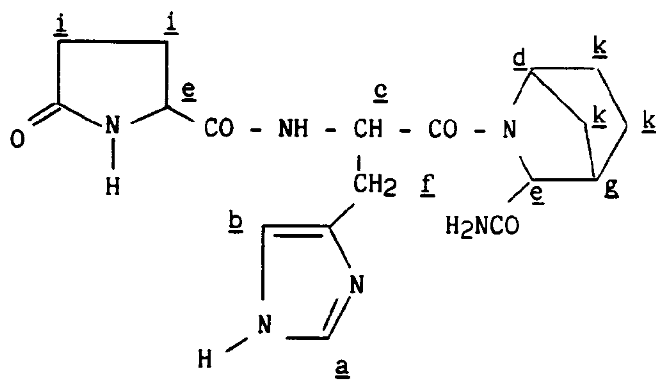

a δ = 7,65 ppm (1H,s)
b δ = 6,95 ppm (1H,s)
c δ = 4,75 ppm (1H,m)
d δ = 4,55 ppm (1H,m)
e δ = 4,05 ppm (2H,m)
f δ entre 3,2 et 2,8 ppm (2H,m)
g δ = 2,7 ppm (1H,m)
i δ entre 2,4 et 1,7 ppm (4H,m)
k δ entre 1,7 et 1,3 ppm (6H,m)

**EXEMPLE 10 : (S)HomoPyroGlu-(S)His-(1S,3S,4R)ABH-NH$_2$**

En procédant comme dans l'exemple 9, mais en remplaçant au stade H le Z(S)PyroGlu-OH par le (S)HomoPyroGlu-OH, on obtient le produit attendu.

Résonance magnétique nucléaire du proton (DMSO $d_6$) :

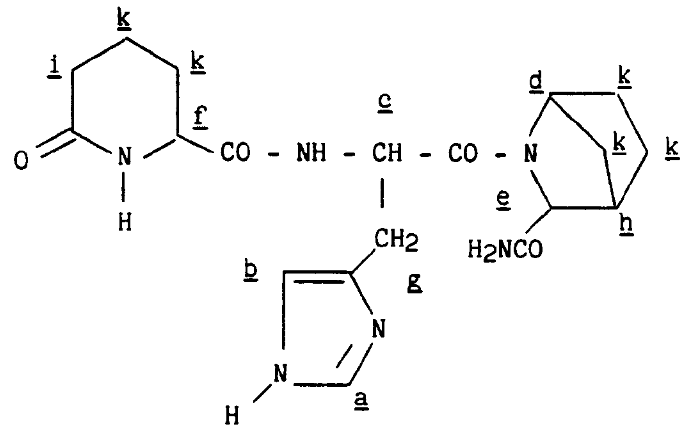

a δ = 7,7 ppm (1H,s)
b δ = 6,95 ppm (1H,s)
c δ = 4,75 ppm (1H,m)
d δ = 4,55 ppm (1H,m)
e δ = 4,00 ppm (1H,d)
f δ = 3,90 ppm (1H,m)
g δ entre 3,1 et 2,8 ppm (2H,m)
h δ = 2,75 ppm (1H,m)
i δ = 2,1 ppm (2H,t)
k δ entre 2,0 et 1,2 ppm (10H,m)

**EXEMPLE 11 : (S)HomoPyroGlu-(S)($N^{\tau}$-Me)His-(1S,3S,4R)ABH-$NH_2$**

En procédant comme dans l'exemple 7, mais en remplaçant au stade F la tBOC-(S)($N^{\pi}$-Me)His-OH par la tBOC-(S)($N^{\tau}$-Me)His-OH, on obtient le produit attendu.

Résonance magnétique nucléaire du proton ($CDCl_3$) :

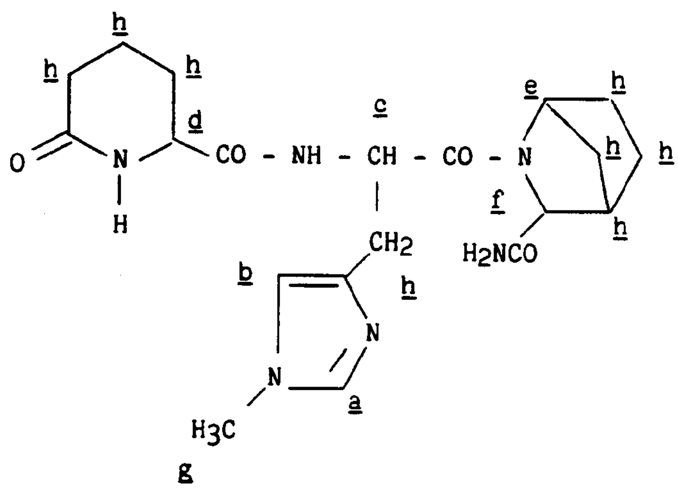

a δ = 7,3 ppm (1H,s)
b δ = 6,7 ppm (1H,s)
c δ = 4,9 ppm (1H,m)
d δ = 4,5 ppm (1H,m)
e δ = 4,3 ppm (1H,m)
f δ = 4,0 ppm (1H,m)
g δ = 3,6 ppm (3H,s)
h δ entre 3,3 et 1,5 ppm (15H,m)

**EXEMPLE 12 : (S)PyroGlu-(S)His-(1R,3R,4S)ABH-$NH_2$**

En procédant comme dans l'exemple 9, mais en remplaçant au stade F, le (1S,3S,4R)ABH-$NH_2$ par le (1R,3R,4S)ABH-$NH_2$ obtenu au stade précédent, on obtient le produit attendu.

**EXEMPLE 13 : OTh-(S)($N^{\tau}$-Me)His-(1S,3S,4R)ABH-$NH_2$**

En procédant comme dans l'exemple 5, mais en remplaçant au stade F la tBOC-(S)Leu-OH par la tBOC-(S)($N^{\tau}$-Me)His-OH et au stade H le Z-(S)PyroGlu-OH par le Z-OTh-OH, on obtient le produit attendu.

Rendement : 66 %

Microanalyse élémentaire :

| | C % | H % | N % | S % |
|---|---|---|---|---|
| Calculé | 51,42 | 5,75 | 19,99 | 7,63 |
| Trouvé | 51,33 | 5,76 | 20,09 | 7,98 |

**EXEMPLE 14 : dOPyr-(S)($N^{\tau}$-Me)His-(1S,3S,4R)ABH-$NH_2$**

En procédant a comme dans l'exemple 13, mais en remplaçant au stade H la Z-OTh-OH par le Z-dOPyr-OH, on obtient le produit attendu.

Rendement : 53 %

Résonance magnétique nucléaire du proton (DMSO $d_6$) :

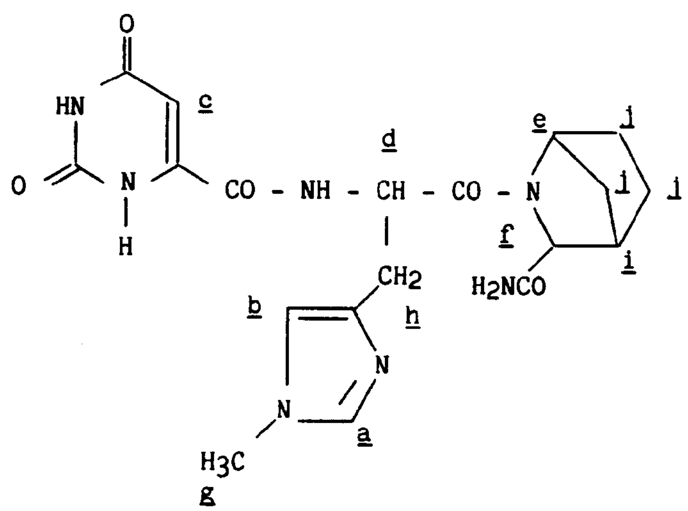

a $\delta$ = 7,4 ppm (1H,s)
b $\delta$ = 6,9 ppm (1H,s)
c $\delta$ = 6,0 ppm (1H,s)
d $\delta$ = 4,8 ppm (1H,m)
e $\delta$ = 4,6 ppm (1H,m)
f $\delta$ = 4,0 ppm (1H,d)
g $\delta$ = 3,6 ppm (3H,s)
h $\delta$ = 2,9 ppm (2H,m)
i $\delta$ = 2,7 ppm (1H,m)
j $\delta \simeq$ 1,5 ppm (6H,m)

**EXEMPLE 15 : OAz-(S)Leu-(1S,3S,4R)ABH-$NH_2$**

En procédant comme dans l'exemple 5, mais en remplaçant au stade H le Z(S)PyroGlu-OH par le Z-OAz-OH préparé selon K. TANAKA, S. YOSHIFUZI et Y. NITTA, Hétérocycles, 1986, 24 (9), 2539, on obtient le produit attendu.

Rendement : 43 %

Résonance magnétique nucléaire du proton (DMSO $d_6$) :

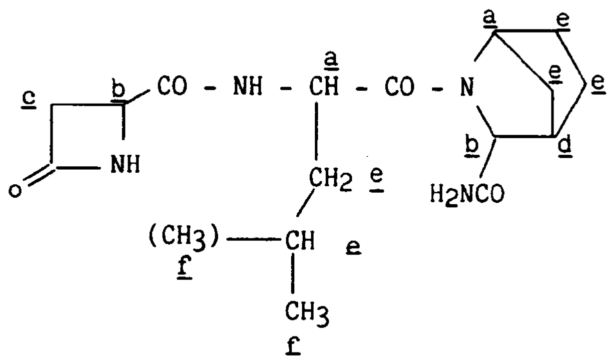

a δ = 4,5 ppm (2H,m)
b δ = 4,0 ppm (2H,m)
c = entre 3,1 et 2,6 ppm (2H,m)
d δ = 2,7 ppm (1H,m)
e δ = 1,6 ppm (9H,m)
f δ = 0,9 ppm (6H,m)

**EXEMPLE 16 : OiQ-(S)Leu-(1S,3S,4R)ABH-$NH_2$**

En procédant comme dans l'exemple 5, mais en remplaçant au stade H le Z(S)PyroGlu-OH par le Z-OiQ-OH préparé selon H. MAEDA et M. SUZUKI, Chem. Pharm. Bull. 1988, 36(1), 190, on obtient le produit attendu.

**EXEMPLE 17 : (S)HomoPyroGlu-(S)Leu-MIABO-$NH_2$ "isomère α"**

En procédant comme dans l'exemple 8, mais en remplaçant au stade A l'ABH-OH "isomères αδ" par le MIABO-OH "isomères αβ" obtenu dans la préparation A, on obtient le produit attendu.

**EXEMPLE 18 : (S)PyroGlu-(S)($N^{\pi}$-Me)His-dMABO-$NH_2$ "isomère α"**

En procédant comme dans l'exemple 6, mais en remplaçant au stade A l'ABH-OH "isomères αδ" par le dMABO-OH "isomères αβ" obtenu dans la préparation B, on obtient le produit attendu.

**EXEMPLE 19 : (S)PyroGlu-(S)Leu-dMABO-$NH_2$ "isomère α"**

En procédant comme dans l'exemple 5, mais en remplaçant au stade A l'ABH-OH "isomères αδ" par le dMABO-OH "isomères αβ" obtenu dans la préparation B, on obtient le produit attendu.

Rendement : 53 %

Microanalyse élémentaire :

| | C % | H % | N % |
|---|---|---|---|
| Calculé | 62,05 | 8,43 | 13,78 |
| Trouvé | 62,20 | 8,66 | 13,33 |

**EXEMPLE 20 : (S)PyroGlu-(S) Leu-MIABO-NH2 "isomère α"**

En procédant comme dans l'exemple 5, mais en remplaçant au stade A l'ABH-OH "isomères αδ" par le MIABO-OH "isomères αβ" obtenu dans la préparation A, on obtient le produit attendu.

**EXEMPLE 21 : OiQ-(S)($N^{\tau}$-Me)His-(1S,3S,4R)ABH-$NH_2$**

En procédant comme dans l'exemple 5, mais en remplaçant au stade F la tBOC-(S)Leu-OH par la tBOC-(S)($N^{\tau}$-Me)His-OH et au stade H le Z-(S)PyroGlu-OH par le Z-OiQ-OH préparé selon M.MAEDA et M.SUZUKI, Chem.Pharm.Bull.1988, 36 (1), 190, on obtient le produit attendu.

Microanalyse élémentaire :

| | C % | H % | N % |
|---|---|---|---|
| Calculé | 62,06 | 6,08 | 18,09 |
| Trouvé | 61,71 | 6,55 | 18,44 |

**EXEMPLE 22 : (S)OAz-(S)($N^{\tau}$-Me)His-(1S,3S,4R)ABH-$NH_2$**

En procédant comme dans l'exemple 5, mais en remplaçant au stade F la tBOC-(S)Leu-OH par la tBOC-(S)($N^{\tau}$-Me)His-OH et au stade H le Z(S)PyroGlu-OH par le Z-OAz-OH préparé selon K.TANAKA, S.YOSHIFUZI et Y.NITTA, Héterocycles, 1986, 24 (9), 2539, on obtient le produit attendu.

Rendemnent : 51 %

Microanalyse élémentaire :

| | C % | H % | N % |
|---|---|---|---|
| Calculé | 55,66 | 6,23 | 21,64 |
| Trouvé | 56,03 | 6,51 | 21,61 |

**EXEMPLE 23 : (S)OAz-(S)His-(1S,3S,4R)ABH-$NH_2$**

En procédant comme dans l'exemple 5, mais en remplaçant au stade F la tBOC-(S)Leu-OH par la tBOC-(S)His-OH et au stade H le Z(S)PyroGlu-OH par le Z-OAz-OH préparé selon K.TANAKA, S.YOSHIFUZI et Y.NITTA, Hétérocycles, 1986, 24 (9), 2549, on obtient le produit attendu.

Rendement : 61 %

Résonance magnétique nucléaire du proton (DMSO $d_6$)

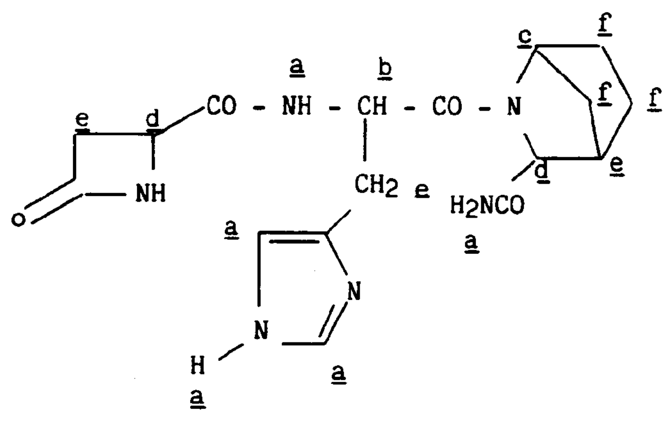

a δ entre 8,2 et 7,0 ppm (6H,m)
b δ = 4,8 ppm (1H,m)
c δ = 4,6 ppm (1H,m)
d δ = 4,0 ppm (2H,m)
e δ = entre 3,1 et 2,5 ppm (5H,m)
f δ ≃ 1,5 ppm (6H,m)

## ETUDE PHARMACOLOGIQUE DES DERIVES DE L'INVENTION

### EXEMPLE 24 : Déficit cholinergique aux barbituriques chez le Rat

Chez le Rat, la narcose au pentobarbital (60 mg/kg ip) entraîne un hypofonctionnement marqué de la neurotransmission cholinergique qui se manifeste notamment par la baisse (- 48 %) de la capture synaptosomale de choline à haute affinité (HACU) mesurée après 20 minutes de narcose. Cette capture sodium-dépendante constitue normalement le facteur limitant de la synthèse d'acétylcholine. L'administration de TRH (10 mg/kg ip) simultanément à celle de pentobarbital n'inhibe pas la narcose mais s'oppose (- 35 %) à la baisse d'HACU induit par le barbiturique.

Dans les mêmes conditions et à la dose de 10 mg/kg ip, le composé de l'exemple 1 inhibe de 66 % la baisse de l'HACU.

### EXEMPLE 25 : Tremblements à l'oxotrémorine chez la Souris

L'administration d'oxotrémorine (0,5 mg/kg ip) chez la Souris entraîne des symptomes neurologiques muscariniques d'origine centrale dont les tremblements en sont l'expression la plus marquée. Ceux-ci atteignent leur maximum d'intensité après 15 minutes et disparaissent en une heure. L'administration de TRH (10 mg/kg ip) 30 minutes avant l'oxotrémorine entraîne une potentialisation (+ 90 %) des tremblements mesurés à l'acmée d'action de l'agoniste muscarinique. Mais cet effet n'apparaît plus à la dose de 3 mg/kg ip.

Dans les mêmes conditions, le composé de l'exemple 1 exerce la même potentialisation (+ 90 %) à la dose de 10 mg/kgip sans induire de tremblements par lui-même.

Cet effet est encore marqué (+ 60 %) et significatif à la dose de 3 mg/kg ip. Il se manifeste encore 60 minutes après l'administration d'oxotrémorine alors que les animaux témoins ne présentent plus de tremblements.

Par voie orale, à la dose de 10 mg/kg, le composé de l'exemple 1 exerce un effet identique à celui observé par voie IP à la dose de 3 mg/kg. La TRH administrée par voie orale n'exerce aucun effet potentialisateur.

L'origine de cet effet potentialisateur des actions centrales de l'oxotrémorine apparaît différente dans nos conditions expérimentales, entre la TRH et le composé pris en exemple. En effet, en présence d'une très faible dose (0,01 mg/kg) de scopolamine qui ne s'oppose pas aux tremblements chez les animaux témoins, l'effet potentialisateur du composé de l'exemple 1 disparaît alors que celui de la TRH subsiste.

Le composé de l'exemple 1 apparaît donc comme un agent facilitateur cholinergique tandis que la TRH pourrait exercer cet effet potentialisateur par agonisme dopaminergique.

### EXEMPLE 26 : Interaction avec la clonidine

Chez la Souris, la clonidine, agoniste $\alpha_2$ central, entraîne un effet sédatif hypométabolisant cérébral qui conduit à une augmentation (+ 60 %) du temps de survie cérébrale lors d'arrêt cardiaque brutal provoqué par l'injection intraveineuse d'une dose massive de $MgCl_2$.

Administrée simultanément à la clonidine, la TRH antagonise l'effet de l'agoniste $\alpha_2$ (1 mg/kg ip : - 20 %; 3 mg/kg ip : - 80 %) sans avoir d'effet propre dans le test utilisé.

Dans les mêmes conditions, le composé de l'exemple 1 antagonise l'effet sédatif de la clonidine (0,3 mg/kg ip : -20% ; 1 mg/kg ip : -70%).

Le composé de l'exemple 1 facilite donc la neurotransmission noradrénergique et s'oppose à la sédation provoquée par l'inhibition de cette neurotransmission.

## Revendications

### Revendications pour les Etats contractants suivants : AT, BE, CH, DE, DK, FR, GB, IT, LI, LU, NL, SE

**1.** Composés de formule générale (I) :

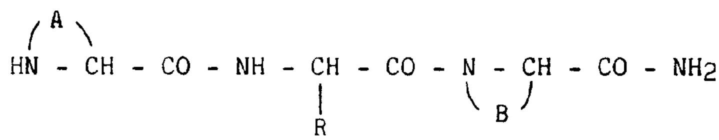

(I)

dans laquelle :

A représente avec les atomes d'azote et de carbone avec lesquels il est lié :

- un groupement oxo-2 pyrrolidinyle-5,
- un groupement oxo-2 pipéridinyle-6,
- un groupement dioxo-2,6 tétrahydro-1,2,3,6 pyrimidinyle-4,
- un groupement oxo-2 thiazolidinyle-4,
- un groupement oxo-2 azetidinyle-4 ,
- un groupement oxo-1 tétrahydro-1,2,3,4 isoquinolyle-3,

B représente avec les atomes d'azote et de carbone avec lesquels il est lié une structure polycyclique saturée choisie parmi l'aza-2 bicyclo [2.2.1] heptane, les (dialkyl ($C_1$-$C_6$) linéaires ou ramifiés)-1,4 aza-2 bicyclo [2.2.2] octane,

R représente un atome d'hydrogène, un groupement alkyle inférieur linéaire ou ramifié, un groupement (imidazolyl-4) méthyle éventuellement substitué sur l'un des atomes d'azote par un radical alkyle inférieur, linéaire ou ramifié,
le terme inférieur indiquant que les groupements ainsi qualifiés comptent de 1 à 6 atomes de carbone,

leurs énantiomères, diastéréoisomères et épimères, ainsi que leurs sels d'addition à un acide pharmaceutiquement acceptable.

**2.** Composés selon la revendication 1 tels que B forme avec les atomes d'azote et de carbone avec lesquels il est lié un cycle aza-2 bicyclo [2.2.1] heptane, leurs énantiomères, diastéréoisomères et épimères ainsi que leurs sels d'addition à un acide pharmaceutiquement acceptable.

**3.** Composés selon la revendication 1 tels que B forme avec les atomes d'azote et de carbone avec lesquels il est lié un cycle dialkyl-1,4 aza-2 bicyclo [2.2.2] octane, leurs énantiomères, diastéréoisomères et épimères ainsi leurs sels d'addition à un acide pharmaceutiquement acceptable.

**4.** Composés selon les revendications 1 et 3 tels que B forme avec les atomes d'azote et de carbone avec lesquels il est lié un cycle diméthyl-1,4 aza-2 bicyclo [2.2.2] octane, leurs énantiomères, diastéréoisomères et épimères ainsi que leurs sels d'addition à un acide pharmaceutiquement acceptable.

**5.** Composés selon les revendications 1 et 3 tels que B forme avec les atomes d'azote et de carbone avec lesquels il est lié un cycle méthyl-4 isopropyl-1 aza-2 bicyclo [2.2.2] octane, leurs énantiomères, diastéréoisomères et épimères ainsi que leurs sels d'addition à un acide pharmaceutiquement acceptable.

**6.** Composé selon les revendications 1 et 2 qui est le PyroGlu-($N^{\tau}$-Me)His-ABH-$NH_2$, ses énantiomères, diastéréoisomères et épimères ainsi que ses sels d'addition à un acide pharmaceutiquement acceptable, PyroGlu représentant le radical pyrrolidone-2 carbonyle-5, ($N^{\tau}$-Me)His représentant le radical méthyl-1 histidyle et ABH le radical aza-2 carbonyl-3 bicyclo [2.2.1] heptane.

**7.** Composé selon les revendications 1 et 2 qui est le PyroGlu-Leu-ABH-$NH_2$ ses diastéréoisomères, énantiomères et épimères ainsi que ses sels d'addition à un acide pharmaceutiquement acceptable, PyroGlu représentant le radical pyrolidone-2 carbonyle-5, Leu représentant le radical leucyle et ABH le radical aza-2 carbonyl-3 bicyclo [2.2.1] heptane.

**8.** Composé selon les revendications 1 et 2 qui est le PyroGlu-($N^{\pi}$-Me)His-ABH-$NH_2$ ses diastéréoisomères, énantiomères et épimères ainsi que ses sels d'addition à un acide pharmaceutiquement acceptable, PyroGlu représentant le radical pyrrolidone-2 carbonyle-5, ($N^{\pi}$-Me)His le radical méthyl-3 histydile et ABH le radical aza-2 carbonyl-3 bicyclo [2.2.1] heptane.

**9.** Composé selon les revendications 1 et 2 qui est le HomoPyroGlu-($N^{\pi}$-Me)His-ABH-$NH_2$ ses diastétéoisomères, énantiomères et épimères ainsi que ses sels d'addition à un acide pharmaceutiquement acceptable, HomoPyroGlu représentant le radical pipéridinone-2 carbonyle-6, ($N^{\pi}$-Me)His le radical méthyl-3

histidyle et ABH le radical aza-2 carbonyl-3 bicyclo [2.2.1] heptane.

**10.** Composé selon les revendications 1 et 2 qui est le HomoPyroGlu-Leu-ABH-$NH_2$ ses diastéréoisomères, énantiomères et épimères ainsi que ses sels d'addition à un acide pharmaceutiquement acceptable, HomoPyroGlu représentant le radical pipéridinone-2 carbonyle-6, Leu représentant le radical leucyle et ABH le radical aza-2 carbonyl-3 bicyclo [2.2.1] heptane.

**11.** Composé selon les revendications 1 et 2 qui est le PyroGlu-His-ABH-$NH_2$ ses diastéréoisomères, énantiomères et épimères ainsi que ses sels d'addition à un acide pharmaceutiquement acceptable, PyroGlu représentant le radical pyrrolidone-2 carbonyle-5, His représentant le radical histidyle et ABH le radical aza-2 carbonyl-3 bicyclo [2.2.1] heptane.

**12.** Composé selon les revendications 1 et 2 qui est le HomoPyroGlu-His-ABH-$NH_2$ ses diastéréoisomères, énantiomères et épimères ainsi que ses sels d'addition à un acide pharmaceutiquement acceptable, HomoPyroGlu représentant le radical pipéridinone-2 carbonyle-6, His le radical histidyle et ABH le radical aza-2 carbonyle-3 bicyclo [2.2.1] heptane.

**13.** Composé selon les revendications 1 et 2 qui est le HomoPyroGlu-($N^{\tau}$-Me)His-ABH-$NH_2$ ses diastéréoisomères, énantiomères et épimères ainsi que ses sels d'addition à un acide pharmaceutiquement acceptable, HomoPyroGlu représentant le radical pipéridinone-2 carbonyle-6, ($N^{\tau}$-Me)His le radical méthyl-1 histidyle et ABH le radical aza-2 carbonyl-3 bicyclo [2.2.1] heptane.

**14.** Composé selon les revendications 1, 3 et 5 qui est le HomoPyroGlu-Leu-MIABO-$NH_2$ ses diastéréoisomères, énantiomères et épimères ainsi que ses sels d'addition à un acide pharmaceutiquement acceptable, HomoPyroGlu représentant le radical pipéridinone-2 carbonyle-6, Leu représentant le radical leucyle, MIABO le radical méthyl-4 isopropyl-1 aza-2 carbonyl-3 bicyclo [2.2.2] octane.

**15.** Composé selon les revendications 1, 3 et 4 qui est le PyroGlu-($N^{\pi}$-Me)His-dMABO-$NH_2$ ses diastéréoisomères, énantiomères et épimères ainsi que ses sels d'addition à un acide pharmaceutiquement acceptable, PyroGlu représentant le radical pyrolidinone-2 carbonyle-5 ($N^{\pi}$-Me)His le radical méthyl-1 histidyle et dMABO le radical diméthyl-1,4 aza-2 carbonyl-3 bicyclo [2.2.2] octane.

**16.** Procédé de préparation des dérivés de formule (I) caractérisé en ce que l'on protège la fonction amine d'un amino-acide de formule (II) dont on a éventuellement séparé les isomères par une technique classique de séparation :

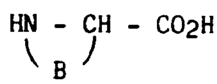

(II)

dans laquelle B a la même signification que dans la formule (I), par un radical protecteur (P) tel que le tert.butoxycarbonyle (tBOC), ou le benzyloxycarbonyle (Z) sous l'action d'un réactif approprié pour conduire à un dérivé de formule (III) :

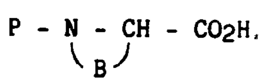

(III)

dans laquelle B et P ont la même signification que précedemment,
sur lequel on fait réagir, à une température comprise entre -15 et 0°C, en présence de triéthylamine, le chloroformiate d'éthyle puis l'ammoniaque,
pour conduire à un dérivé de formule (IV) :,

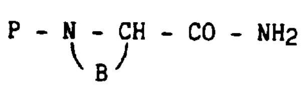

(IV)

dans laquelle B et P ont la même signification que précedemment,
que l'on déprotège par un procédé approprié comme par exemple par action de l'acide chlorhydrique gazeux dans un solvant anhydre tel que le dioxanne ou l'acétate d'éthyle dans le cas où P = t.BOC ou par hydrogénation catalytique dans le cas où P = Z,
pour conduire à un dérivé de formule (V) :

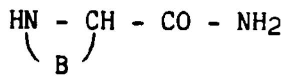

(V)

dans laquelle B a la même signification que dans la formule (I),
dont on sépare, si on le souhaite, les isomères par une technique classique de séparation,
qui est couplé avec un deuxième amino-acide protégé de formule (VI) :

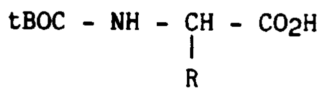

(VI)

dans laquelle R a la même signification que dans la formule (I),
pour conduire à un dérivé de formule (VII) :

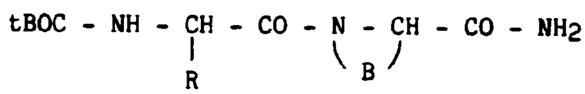

(VII)

dans laquelle R et B ont la même signification que dans la formule (I) dont on sépare, si on le souhaite, les diastéréoisomères ou énantiomères par une technique classique de séparation,
que l'on déprotège ensuite par action de l'acide chlorhydrique gazeux dans un solvant anhydre comme par exemple le dioxanne ou l'acétate d'éthyle pour conduire à un dérivé de formule (VIII) :

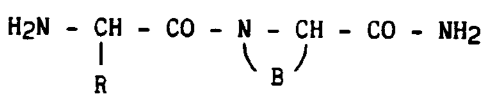

(VIII)

dans laquelle R et B ont la même signification que dans la formule (I),
qui est couplé avec un troisième amino-acide éventuellement protégé de formule (IX) :

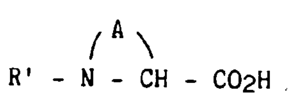

(IX)

dans laquelle R' est un hydrogène, ou un groupement protecteur tel que par exemple un benzyloxycarbonyle (Z),
ou avec un ester activé de cet amino-acide éventuellement protégé, pour conduire :

- ou bien à un dérivé de formule (I) dans le cas où R' est un hydrogène, qui est, si on le désire transformé en son sel d'addition à un acide pharmaceutiquement acceptable,
- ou bien à un dérivé de formule (X) :

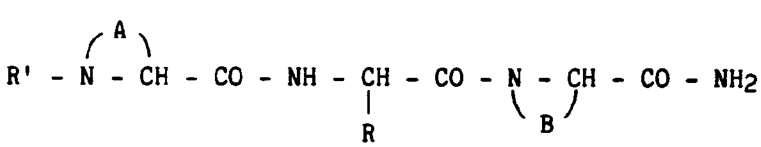

(X)

dans le cas où R' est un groupement protecteur, R et B ayant la même signification que dans la formule (I),

qui est déprotégé par une technique de déprotection comme par exemple une hydrogénation catalytique pour conduire à un dérivé de formule (I), qui est, si on le désire transformé en son sel d'addition à un acide pharmaceutiquement acceptable, ou séparé en ses isomères selon une technique classique de séparation que l'on salifie si on le désire par un acide pharmaceutiquement acceptable.

**17.** Composés de formule (II) selon la revendication 16, qui sont les acides dialkyl-1,4 aza-2 bicyclo [2.2.2] octane carboxyliques-3, utiles dans la préparation des composés de formule (I).

**18.** Composés de formule (III) selon la revendication 16, utiles dans la préparation des composés de formule (I).

**19.** Composés de formule (IV) selon la revendication 16, utilies dans la préparation des composés de formule (I).

**20.** Composés de formule (V) selon la revendication 16, utiles dans la préparation des composés de formule (I).

**21.** Composés de formule (VII) selon la revendication 16, utiles dans la préparation des composés de formule (I).

**22.** Composés de formule (VIII) selon la revendication 16, utiles dans la préparation des composés de formule (I).

**23.** Compositions pharmaceutiques contenant comme principe actif au moins un composé selon l'une quelconque des revendications 1 à 15, seul ou en combinaison avec un ou plusieurs excipients ou véhicules inertes, non toxiques, pharmaceutiquement acceptables.

**24.** Compositions pharmaceutiques selon la revendication 23 contenant au moins un principe actif selon l'une des revendications 1 à 25 utiles pour le traitement des désordres constitutifs et des troubles neurocomportementaux associés au vieillissement et aux maladies dégénératives du système nerveux, aigües ou chroniques comme la maladie d'Alzheimer, l'accident vasculaire cérébral, le traumatisme spinal ou la sclérose amyotrophique latérale.

**Revendications pour l'Etat contractant suivant: GR**

**1.** Procédé de préparation des composés de formule générale (I) :

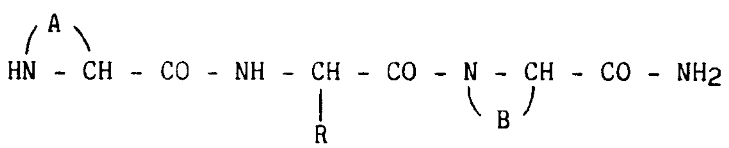

(I)

dans laquelle :

A représente avec les atomes d'azote et de carbone avec lesquels il est lié :
- un groupement oxo-2 pyrrolidinyle-5,
- un groupement oxo-2 pipéridinyle-6,
- un groupement dioxo-2,6 tétrahydro-1,2,3,6 pyrimidinyle-4,
- un groupement oxo-2 thiazolidinyle-4,
- un groupement oxo-2 azetidinyle-4 ,
- un groupement oxo-1 tétrahydro-1,2,3,4 isoquinolyle-3,

B représente avec les atomes d'azote et de carbone avec lesquels il est lié une structure polycyclique saturée choisie parmi l'aza-2 bicyclo [2.2.1] heptane, les (dialkyl ($C_1$-$C_6$) linéaires ou ramifiés)-1,4 aza-2 bicyclo [2.2.2] octane,

R représente un atome d'hydrogène, un groupement alkyle inférieur linéaire ou ramifié, un groupement (imidazolyl-4) méthyle éventuellement substitué sur l'un des atomes d'azote par un radical alkyle inférieur, linéaire ou ramifié,

le terme inférieur indiquant que les groupements ainsi qualifiés comptent de 1 à 6 atomes de carbone, leurs énantiomères, diastéréoisomères et épimères, ainsi que leurs sels d'addition à une acide pharmaceutiquement acceptable,
caractérisé en ce que l'on protège la fonction amine d'un amino-acide de formule (II) dont on a éventuellement séparé les isomères par une technique classique de séparation :

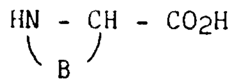

(II)

dans laquelle B a la même signification que dans la formule (I), par un radical protecteur (P) tel que le tert.butoxycarbonyle (tBOC), ou le benzyloxycarbonyle (Z) sous l'action d'un réactif approprié pour conduire à un dérivé de formule (III) :

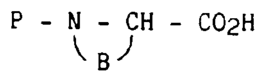

(III)

dans laquelle B et P ont la même signification que précedemment,
sur lequel on fait réagir, à une température comprise entre -15 et 0°C, en présence de triéthylamine, le chloroformiate d'éthyle puis l'ammoniaque,
pour conduire à un dérivé de formule (IV) :

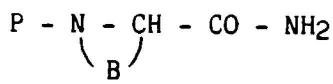

(IV)

dans laquelle B et P ont la même signification que précedemment,
que l'on déprotège par un procédé approprié comme par exemple par action de l'acide chlorhydrique gazeux dans un solvant anhydre tel que le dioxanne ou l'acétate d'éthyle dans le cas où P = t.BOC ou par hydrogénation catalytique dans le cas où P = Z,
pour conduire à un dérivé de formule (V) :

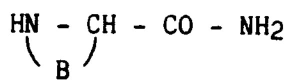

(V)

dans laquelle B a la même signification que dans la formule (I),
dont on sépare, si on le souhaite, les isomères par une technique classique de séparation,
qui est couplé avec un deuxième amino-acide protégé de formule (VI) :

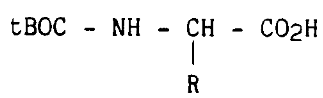

(VI)

dans laquelle R a la même signification que dans la formule (I),
pour conduire à un dérivé de formule (VII) :

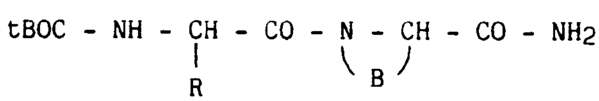

(VII)

dans laquelle R et B ont la même signification que dans la formule (I) dont on sépare, si on le souhaite, les diastéréoisomères ou énantiomères par une technique classique de séparation,

que l'on déprotège ensuite par action de l'acide chlorhydrique gazeux dans un solvant anhydre comme par exemple le dioxanne ou l'acétate d'éthyle pour conduire à un dérivé de formule (VIII) :

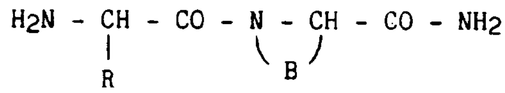

(VIII)

dans laquelle R et B ont la même signification que dans la formule (I),
qui est couplé avec un troisième amino-acide éventuellement protégé de formule (IX) :

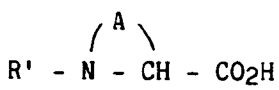

(IX)

dans laquelle R' est un hydrogène, ou un groupement protecteur tel que par exemple un benzyloxycarbonyle (Z),
ou avec un ester activé de cet amino-acid éventuellement protégé, pour conduire :

- ou bien à un dérivé de formule (I) dans le cas où R' est un hydrogène, qui est, si on le désire transformé en son sel d'addition à un acide pharmaceutiquement acceptable,
- ou bien à un dérivé de formule (X) :

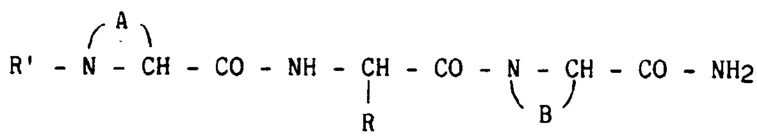

(X)

dans le cas où R' est un groupement protecteur, R et B ayant la même signification que dans la formule (I), qui est déprotégé par une technique de déprotection comme par exemple une hydrogénation catalytique pour conduire à un dérivé de formule (I),
qui est, si on le désire transformé en son sel d'addition à un acide pharmaceutiquement acceptable, ou séparé en ses isomères selon une technique classique de séparation que l'on salifie si on le désire par un acide pharmaceutiquement acceptable.

**2.** Procédé de préparation selon la revendication 1 de composés tels que B forme avec les atomes d'azote et de carbone avec lesquels il est lié un cycle aza-2 bicyclo [2.2.1] heptane, leurs énantiomères, diastéréoisomères et épimères ainsi que leurs sels d'addition à un acide pharmaceutiquement acceptable.

**3.** Procédé de préparation selon la revendication 1 de composés tels que B forme avec les atomes d'azote et de carbone avec lesquels il est lié un cycle dialkyl-1,4 aza-2 bicyclo [2.2.2] octane, leurs énantiomères, diastéréoisomères et épimères ainsi leurs sels d'addition à un acide pharmaceutiquement acceptable.

**4.** Procédé de préparation selon les revendications 1 et 3 de composés tels que B forme avec les atomes d'azote et de carbone avec lesquels il est lié un cycle diméthyl-1,4 aza-2 bicyclo [2.2.2] octane, leurs énantiomères, diastéréoisomères et épimères ainsi que leurs sels d'addition à un acide pharmaceutiquement acceptable.

**5.** Procédé de préparation selon les revendications 1 et 3 de composés tels que B forme avec les atomes d'azote et de carbone avec lesquels il est lié un cycle méthyl-4 isopropyl-1 aza-2 bicyclo [2.2.2] octane, leurs énantiomères, diastéréoisomères et épimères ainsi que leurs sels d'addition à un acide pharmaceutiquement acceptable.

**6.** Procédé de préparation selon les revendications 1 et 2 du composé qui est le PyroGlu-($N^{\tau}$-Me)His-ABH-$NH_2$, ses énantiomères, diastéréoisomères et épimères ainsi que ses sels d'addition à un acide pharmaceutiquement acceptable, PyroGlu représentant le radical pyrrolidone-2 carbonyle-5, ($N^{\tau}$-Me)His représentant le radical méthyl-1 histidyle et ABH le radical aza-2 carbonyl-3 bicyclo [2.2.1] heptane.

**7.** Procédé de préparation selon les revendications 1 et 2 du composé qui est le PyroGlu-Leu-ABH-$NH_2$ ses diastéréoisomères, énantiomères et épimères ainsi que ses sels d'addition à un acide pharmaceutiquement acceptable, PyroGlu représentant le radical pyrolidone-2 carbonyle-5, Leu représentant le radical leucyle et ABH le radical aza-2 carbonyl-3 bicyclo [2.2.1] heptane.

**8.** Procédé de préparation selon les revendications 1 et 2 du composé qui est le PyroGlu-($N^{\pi}$-Me)His-ABH-$NH_2$ ses diastéréoisomères, énantiomères et épimères ainsi que ses sels d'addition à un acide pharmaceutiquement acceptable, PyroGlu représentant le radical pyrrolidone-2 carbonyle-5, ($N^{\pi}$-Me)His le radical méthyl-3 histydile et ABH le radical aza-2 carbonyl-3 bicyclo [2.2.1] heptane.

**9.** Procédé de préparation selon les revendications 1 et 2 du composé qui est le HomoPyroGlu-($N^{\pi}$-Me)His-ABH-$NH_2$ ses diastéréoisomères, énantiomères et épimères ainsi que ses sels d'addition à un acide pharmaceutiquement acceptable, HomoPyroGlu représentant le radical pipéridinone-2 carbonyle-6, ($N^{\pi}$-Me)His le radical méthyl-3 histidyle et ABH le radical aza-2 carbonyl-3 bicyclo [2.2.1] heptane.

**10.** Procédé de préparation selon les revendications 1 et 2 du composé qui est le HomoPyroGlu-Leu-ABH-$NH_2$ ses diastéréoisomères, énantiomères et épimères ainsi que ses sels d'addition à un acide pharmaceutiquement acceptable, HomoPyroGlu représentant le radical pipéridinone-2 carbonyle-6, Leu représentant le radical leucyle et ABH le radical aza-2 carbonyl-3 bicyclo [2.2.1] heptane.

**11.** Procédé de préparation selon les revendications 1 et 2 du composé du est le PyroGlu-His-ABH-$NH_2$ ses diastéréoisomères, énantiomères et épimères ainsi que ses sels d'addition à un acide pharmaceutiquement acceptable, PyroGlu représentant le radical pyrrolidone-2 carbonyle-5, His représentant le radical histidyle et ABH le radical aza-2 carbonyl-3 bicyclo [2.2.1] heptane.

**12.** Procédé de préparation selon les revendications 1 et 2 du composé qui est le HomoPyroGlu-His-ABH-$NH_2$ ses diastéréoisomères, énantiomères et épimères ainsi que ses sels d'addition à un acide pharmaceutiquement acceptable, HomoPyroGlu représentant le radical pipéridinone-2 carbonyl-6, His le radical histidyle et ABH le radical aza-2 carbonyle-3 bicycle [2.2.1] heptane.

**13.** Procédé de préparation selon les revendication 1 et 2 du composé qui est le HomoPyroGlu-($N^{\tau}$-Me)His-ABH-$NH_2$ ses diastéréoisomères, énantiomères et épimères ainsi que ses sels d'addition à un acide pharmaceutiquement acceptable, HomoPyroGlu représentant le radical pipéridinone-2 carbonyle-6, ($N^{\tau}$-Me)His le radical méthyl-1 histidyle et ABH le radical aza-2 carbonyl-3 bicyclo [2.2.1] heptane.

**14.** Procédé de préparation selon les revendications 1, 3 et 5 du composé qui est le HomoPyroGlu-Leu-MIABO-$NH_2$ ses diastéréoisomères, énantiomères et épimères ainsi que ses sels d'addition à un acide pharmaceutiquement acceptable, HomoPyroGlu représentant le radical pipéridinone-2 carbonyle-6, Leu représentant le radical leucyle, MIABO le radical méthyl-4 isopropyl-1 aza-2 carbonyl-3 bicyclo [2.2.2] octane.

**15.** Procédé de préparation selon les revendications 1, 3 et 4 du composé qui est le PyroGlu-($N^{\pi}$-Me)His-dMABO-$NH_2$ ses diastéréoisomères, énantiomères et épimères ainsi que ses sels d'addition à un acide pharmaceutiquement acceptable, PyroGlu représentant le radical pyrolidinone-2 carbonyle-5 ($N^{\pi}$-Me)His le radical méthyl-1 histidyle et dMABO le radical diméthyl-1,4 aza-2 carbonyl-3 bicyclo [2.2.2] octane.

**16.** Procédé de préparation selon la revendication 1 des composés de formule (II) utiles dans la préparation des composés de formule (I).

**17.** Procédé de préparation selon la revendication 1 des composés de formule (III) utiles dans la préparation des composés de formule (I).

**18.** Procédé de préparation selon la revendication 1 des composés de formule (IV) utiles dans la préparation des composés de formule (I).

**19.** Procédé de préparation selon la revendication 1 des composés de formule (V) utiles dans la préparation des composés de formule (I).

**20.** Procédé de préparation selon la revendication 1 des composés de formule (VII) utiles dans la préparation des composés de formule (I).

**21.** Procédé de préparation selon la revendication 1 des composés de formule (VIII) utiles dans la préparation des composés de formule (I).

**Revendications pour l'Etat contractant suivant : ES**

**1.** Procédé de préparation des composés de formule générale (I) :

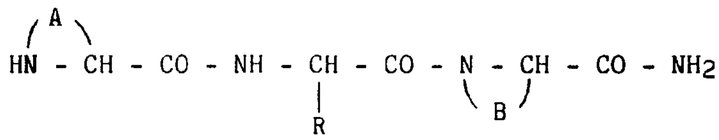

(I)

dans laquelle

A représente avec les atomes d'azote et de carbone avec lesquels il est lié :
- un groupement oxo-2 pyrrolidinyle-5,
- un groupement oxo-2 pipéridinyle-6,
- un groupement dioxo-2,6 tétrahydro-1,2,3,6 pyrimidinyle-4,
- un groupement oxo-2 thiazolidinyle-4,
- un groupement oxo-2 azetidinyle-4 ,
- un groupement oxo-1 tétrahydro-1,2,3,4 isoquinolyle-3,

B représente avec les atomes d'azote et de carbone avec lesquels il est lié une structure polycyclique saturée choisie parmi l'aza-2 bicyclo [2.2.1] heptane, les (dialkyl ($C_1$-$C_6$) linéaires ou ramifiés)-1,4 aza-2 bicyclo [2.2.2] octane,

R représente un atome d'hydrogène, un groupement alkyle inférieur linéaire ou ramifié, un groupement (imidazolyl-4) méthyle éventuellement substitué sur l'un des atomes d'azote par un radical alkyle inférieur, linéaire ou ramifié,

le terme inférieur indiquant que les groupements ainsi qualifiés comptent de 1 à 6 atomes de carbone, leurs énantiomères, diastéréoisomères et épimères, ainsi que leurs sels d'addition à un acide pharmaceutiquement acceptable,
caractérisé en ce que l'on protège la fonction amine d'un amino-acide de formule (II) dont on a éventuellement séparé les isomères par une technique classique de séparation :

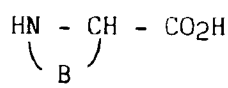

(II)

dans laquelle B a la même signification que dans la formule (I), par un radical protecteur (P) tel que le tert.butoxycarbonyle (tBOC), ou le benzyloxycarbonyle (Z) sous l'action d'un réactif approprié pour conduire à un dérivé de formule (III) :

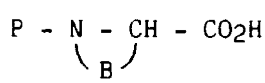

(III)

dans laquelle B et P ont la même signification que précedemment,
sur lequel on fait réagir, à une température comprise entre -15 et 0°C, en présence de triéthylamine, le chloroformiate d'éthyle puis l'ammoniaque,
pour conduire à un dérivé de formule (IV) :

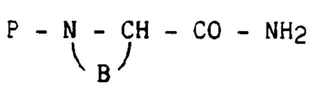

(IV)

dans laquelle B et P ont la même signification que précedemment,
que l'on déprotège par un procédé approprié comme par exemple par action de l'acide chlorhydrique gazeux dans un solvant anhydre tel que le dioxanne ou l'acétate d'éthyle dans le cas où P = t.BOC ou par hydrogénation catalytique dans le cas où P = Z,

pour conduire à un dérivé de formule (V) :

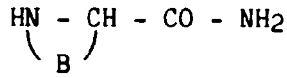

(V)

dans laquelle B a la même signification que dans la formule (I),
dont on sépare, si on le souhaite, les isomères par une technique classique de séparation,
qui est couplé avec un deuxième amino-acide protégé de formule (VI) :

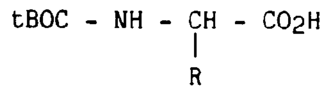

(VI)

dans laquelle R a la même signification que dans la formule (I), pour conduire à un dérivé de formule (VII) :

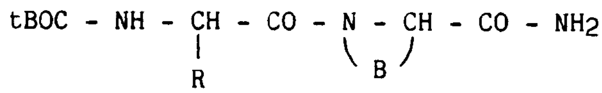

(VII)

dans laquelle R et B ont la même signification que dans la formule (I) dont on sépare, si on le souhaite, les diastéréoisomères ou énantiomères par une technique classique de séparation,
que l'on déprotège ensuite par action de l'acide chlorhydrique gazeux dans un solvant anhydre comme par exemple le dioxanne ou l'acétate d'éthyle pour conduire à un dérivé de formule (VIII) :

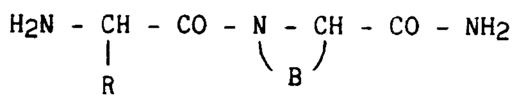

(VIII)

dans laquelle R et B ont la même signification que dans la formule (I),
qui est couplé avec un troisième amino-acide éventuellement protégé de formule (IX) :

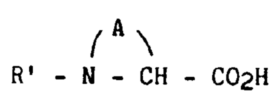

(IX)

dans laquelle R' est un hydrogène, ou un groupement protecteur tel que par exemple un benzyloxycarbonyle (Z),
ou avec un ester activé de cet amino-acid éventuellement protégé, pour conduire :

- ou bien à un dérivé de formule (I) dans le cas où R' est un hydrogène, qui est, si on le désire transformé en son sel d'addition à un acide pharmaceutiquement acceptable,
- ou bien à un dérivé de formule (X) :

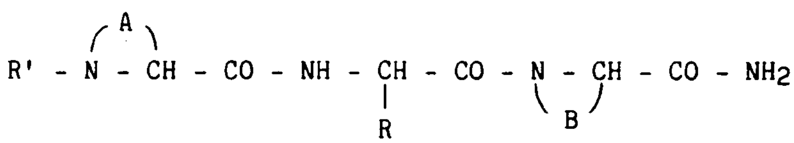

(X)

dans le cas où R' est un groupement protecteur, R et B ayant la même signification que dans la formule (I),
qui est déprotégé par une technique de déprotection comme par exemple une hydrogénation catalytique pour conduire à un dérivé de formule (I),
qui est, si on le désire transformé en son sel d'addition à un acide pharmaceutiquement acceptable, ou séparé en ses isomères selon une technique classique de séparation que l'on salifie si on le désire par

un acide pharmaceutiquement acceptable.

2. Procédé de préparation selon la revendication 1 de composés tels que B forme avec les atomes d'azote et de carbone avec lesquels il est lié un cycle aza-2 bicyclo [2.2.1] heptane, leurs énantiomères, diastéréoisomères et épimères ainsi que leurs sels d'addition à un acide pharmaceutiquement acceptable.

3. Procédé de préparation selon la revendication 1 de composés tels que B forme avec les atomes d'azote et de carbone avec lesquels il est lié un cycle dialkyl-1,4 aza-2 bicyclo [2.2.2] octane, leurs énantiomères, diastéréoisomères et épimères ainsi leurs sels d'addition à un acide pharmaceutiquement acceptable.

4. Procédé de préparation selon les revendications 1 et 3 de composés tels que B forme avec les atomes d'azote et de carbone avec lesquels il est lié un cycle diméthyl-1,4 aza-2 bicyclo [2.2.2] octane, leurs énantiomères, diastéréoisomères et épimères ainsi que leurs sels d'addition à un acide pharmaceutiquement acceptable.

5. Procédé de préparation selon les revendications 1 et 3 de composés tels que B forme avec les atomes d'azote et de carbone avec lesquels il est lié un cycle méthyl-4 isopropyl-1 aza-2 bicyclo [2.2.2] octane, leurs énantiomères, diastéréoisomères et épimères ainsi que leurs sels d'addition à un acide pharmaceutiquement acceptable.

6. Procédé de préparation selon les revendications 1 et 2 du composé qui est le PyroGlu-($N^{\tau}$-Me)His-ABH-$NH_2$, ses énantiomères, diastéréoisomères et épimères ainsi que ses sels d'addition à un acide pharmaceutiquement acceptable, PyroGlu représentant le radical pyrrolidone-2 carbonyle-5, ($N^{\tau}$-Me)His représentant le radical méthyl-1 histidyle et ABH le radical aza-2 carbonyl-3 bicyclo [2.2.1] heptane.

7. Procédé de préparation selon les revendications 1 et 2 du composé qui est le PyroGlu-Leu-ABH-$NH_2$ ses diastéréoisomères, énantiomères et épimères ainsi que ses sels d'addition à un acide pharmaceutiquement acceptable, PyroGlu représentant le radical pyrolidone-2 carbonyle-5, Leu représentant le radical leucyle et ABH le radical aza-2 carbonyl-3 bicyclo [2.2.1] heptane.

8. Procédé de préparation selon les revendications 1 et 2 du composé qui est le PyroGlu-($N^{\Pi}$-Me)His-ABH-$NH_2$ ses diastéréoisomères, énantiomères et épimères ainsi que ses sels d'addition à un acide pharmaceutiquement acceptable, PyroGlu représentant le radical pyrrolidone-2 carbonyle-5, ($N^{\Pi}$-Me)His le radical méthyl-3 histydile et ABH le radical aza-2 carbonyl-3 bicyclo [2.2.1] heptane.

9. Procédé de préparation selon les revendications 1 et 2 du composé qui est le HomoPyroGlu-($N^{\Pi}$-Me)His-ABH-$NH_2$ ses diastéréoisomères, énantiomères et épimères ainsi que ses sels d'addition à un acide pharmaceutiquement acceptable, HomoPyroGlu représentant le radical pipéridinone-2 carbonyle-6, ($N^{\Pi}$-Me)His le radical méthyl-3 histidyle et ABH le radical aza-2 carbonyl-3 bicyclo [2.2.1] heptane.

10. Procédé de préparation selon les revendications 1 et 2 du composé qui est le HomoPyroGlu-Leu-ABH-$NH_2$ ses diastéréoisomères, énantiomères et épimères ainsi que ses sels d'addition à un acide pharmaceutiquement acceptable, HomoPyroGlu représentant le radical pipéridinone-2 carbonyle-6, Leu représentant le radical leucyle et ABH le radical aza-2 carbonyl-3 bicyclo [2.2.1] heptane.

11. Procédé de préparation selon les revendications 1 et 2 du composé du est le PyroGlu-His-ABH-$NH_2$ ses diastéréoisomères, énantiomères et épimères ainsi que ses sels d'addition à un acide pharmaceutiquement acceptable, PyroGlu représentant le radical pyrrolidone-2 carbonyle-5, His représentant le radical histidyle et ABH le radical aza-2 carbonyl-3 bicyclo [2.2.1] heptane.

12. Procédé de préparation selon les revendications 1 et 2 du composé qui est le HomoPyroGlu-His-ABH-$NH_2$ ses diastéréoisomères, énantiomères et épimères ainsi que ses sels d'addition à un acide pharmaceutiquement acceptable, HomoPyroGlu représentant le radical pipéridinone-2 carbonyl-6, His le radical histidyle et ABH le radical aza-2 carbonyle-3 bicycle [2.2.1] heptane.

13. Procédé de préparation selon les revendication 1 et 2 du composé qui est le HomoPyroGlu-($N^{\tau}$-Me)His-ABH-$NH_2$ ses diastéréoisomères, énantiomères et épimères ainsi que ses sels d'addition à un acide pharmaceutiquement acceptable, HomoPyroGlu représentant le radical pipéridinone-2 carbonyle-6, ($N^{\tau}$-Me)His le radical méthyl-1 histidyle et ABH le radical aza-2 carbonyl-3 bicyclo [2.2.1] heptane.

**14.** Procédé de préparation selon les revendications 1, 3 et 5 du composé qui est le HomoPyroGlu-Leu-MIABO-$NH_2$ ses diastéréoisomères, énantiomères et épimères ainsi que ses sels d'addition à un acide pharmaceutiquement acceptable, HomoPyroGlu représentant le radical pipéridinone-2 carbonyle-6, Leu représentant le radical leucyle, MIABO le radical méthyl-4 isopropyl-1 aza-2 carbonyl-3 bicyclo [2.2.2] octane.

**15.** Procédé de préparation selon les revendications 1, 3 et 4 du composé qui est le PyroGlu-($N^{\pi}$-Me)His-dMABO-$NH_2$ ses diastéréoisomères, énantiomères et épimères ainsi que ses sels d'addition à un acide pharmaceutiquement acceptable, PyroGlu représentant le radical pyrolidinone-2 carbonyle-5 ($N^{\pi}$-Me)His le radical méthyl-1 histidyle et dMABO le radical diméthyl-1,4 aza-2 carbonyl-3 bicyclo [2.2.2] octane.

**16.** Procédé de préparation selon la revendication 1 des composés de formule (II) utiles dans la préparation des composés de formule (I).

**17.** Procédé de préparation selon la revendication 1 des composés de formule (III) utiles dans la préparation des composés de formule (I).

**18.** Procédé de préparation selon la revendication 1 des composés de formule (IV) utiles dans la préparation des composés de formule (I).

**19.** Procédé de préparation selon la revendication 1 des composés de formule (V) utiles dans la préparation des composés de formule (I).

**20.** Procédé de préparation selon la revendication 1 des composés de formule (VII) utiles dans la préparation des composés de formule (I).

**21.** Procédé de préparation selon la revendication 1 des composés de formule (VIII) utiles dans la préparation des composés de formule (I).

## Patentansprüche

### Patentansprüche für folgende Vertragsstaaten: AT, BE, CH, DE, DK, FR, GB, IT, LI, LU, NL, SE

**1.** Verbindungen der allgemeinen Formel (I):

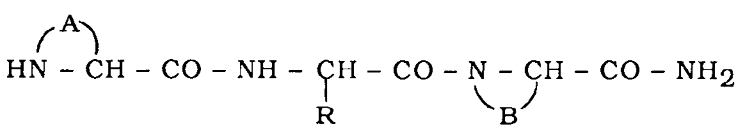

(I)

in der

A zusammen mit dem Stickstoff- und Kohlenstoffatom, an die es gebunden ist,
- eine 2-Oxo-pyrrolidin-5-yl-gruppe,
- eine 2-Oxo-piperidin-6-yl-gruppe,
- eine 2,6-Dioxo-1,2,3,6-tetrahydro-pyrimidin-4-yl-gruppe,
- eine 2-Oxo-thiazolidin-4-yl-gruppe,
- eine 2-Oxo-azetidin-4-yl-gruppe,
- eine 1-Oxo-1,2,3,4-tetrahydro-isochinol-3-yl-gruppe,

B zusammen mit dem Stickstoff- und Kohlenstoffatom, an die es gebunden ist, eine gesättigte polycyclische Struktur ausgewählt aus 2-Aza-bicyclo-[2.2.1]heptan und 1,4-(geradkettige oder verzweigte ($C_1$-$C_6$)-dialkyl)-2-aza-bicyclo[2.2.2]octanen,

R ein Wasserstoffatom, eine geradkettige oder verzweigte Niedrigalkylgruppe, eine (Imidazol-4-yl)-methylgruppe, die gegebenenfalls an einem der Stickstoffatome durch eine geradkettige oder verzweigte Niedrigalkylgruppe substituiert ist,

bedeuten, wobei der Begriff "Niedrig" darauf hinweist, daß die in dieser Weise bezeichneten Gruppen 1 bis 6 Kohlenstoffatome aufweisen. deren Enantiomere, Diastereoisomere und Epimere sowie deren Additionssalze mit einer pharmazeutisch annehmbaren Säure.

2. Verbindungen nach Anspruch 1, worin B mit dem Stickstoff- und dem Kohlenstoffatom, an die es gebunden ist, einen 2-Aza-bicyclo[2.2.1]heptanring bildet, deren Enantiomere, Diastereoisomere und Epimere sowie deren Additionssalze mit einer pharmazeutisch annehmbaren Säure.

3. Verbindungen nach Anspruch 1, worin B zusammen mit dem Stickstoffund dem Kohlenstoffatom, an die es gebunden ist, einen 1,4-Dialkyl-2-aza-bicyclo[2.2.2]octanring bildet, deren Enantiomere, Diastereoisomere und Epimere sowie deren Additionssalze mit einer pharmazeutisch annehmbaren Säure.

4. Verbindungen nach den Ansprüchen 1 und 3, worin B zusammen mit dem Stickstoff- und dem Kohlenstoffatom, an die es gebunden ist, einen 1,4-Dimethyl-2-aza-bicyclo[2.2.2]octanring bildet, deren Enantiomere, Diastereoisomere und Epimere sowie deren Additionssalze mit einer pharmazeutisch annehmbaren Säure.

5. Verbindungen nach den Ansprüchen 1 und 3, worin B zusammen mit dem Stickstoff- und dem Kohlenstoffatom, an die es gebunden ist, einen 4-Methyl- 1-isopropyl-2-aza-bicyclo[2.2.2]octanring bildet, deren Enantiomere, Diastereoisomere und Epimere sowie deren Additionssalze mit einer pharmazeutisch annehmbaren Säure.

6. Verbindung nach den Ansprüchen 1 und 2, nämlich PyroClu-($N^{\tau}$-Me)-His-ABH-$NH_2$, deren Enantiomere, Diastereoisomere und Epimere sowie deren Additionssalze mit einer pharmazeutisch annehmbaren Säure, worin PyroGlu den 2-Pyrrolidon-5-carbonylrest. ($N^{\tau}$-Me)His den 1-Methyl-histidylrest und ABH den 2-Aza-3-carbonyl-bicyclo[2.2.1]heptanrest bedeuten.

7. Verbindung nach den Ansprüchen 1 und 2, nämlich PyroGlu-Leu-ABH- $NH_2$, deren Diastereoisomere, Enantiomere und Epimere sowie deren Additionssalze mit einer pharmazeutisch annehmbaren Säure, worin PyroGlu den 2-Pyrrolidon-5-carbonylrest, Leu den Leucylrest und ABH den 2-Aza-3-carbonyl-bicyclo[2.2.1]heptanrest bedeuten.

8. Verbindung nach den Ansprüchen 1 und 2, nämlich PyroClu-($N^{\tau}$-Me)-His-ABH-$NH_2$, deren Diastereoisomere, Enantiomere und Epimere sowie deren Additionssalze mit einer pharmazeutisch annehmbaren Säure, worin PyroGlu den 2-Pyrrolidon-5-carbonylrest, ($N^{\tau}$-Me)His den 3-Methyl-histidylrest und ABH den 2-Aza-3-carbonyl-bicyclo[2.2.1]heptanrest bedeuten.

9. Verbindung nach den Ansprüchen 1 und 2, nämlich HomoPyroClu-($N^{\pi}$- Me)His-ABH-$NH_2$, deren Diastereoisomere. Enantiomere und Epimere sowie deren Additionssalze mit einer pharmazeutisch annehmbaren Säure, worin HomoPyroGlu den 2-Piperidinon-6-carbonylrest, ($N^{\pi}$-Me)His den 3-Methyl-histidylrest und ABH den 2-Aza-3-carbonyl-bicyclo[2.2.1]heptanrest bedeuten.

10. Verbindung nach den Ansprüchen 1 und 2, nämlich HomoPyroGlu-Leu- ABH-$NH_2$, deren Diastereoisomere, Enantiomere und Epimere sowie deren Additionssalze mit einer pharmazeutisch annehmbaren Säure, worin HomoPyroGlu den 2-Piperidinon-6-carbonylrest, Leu den Leucylrest und ABH den 2-Aza-3-carbonyl-bicyclo[2.2.1]heptanrest bedeuten.

11. Verbindung nach den Ansprüchen 1 und 2, nämlich PyroGluHis-ABH-$NH_2$, deren Diastereoisomere, Enantiomere und Epimere sowie deren Additionssalze mit einer pharmazeutisch annehmbaren Säure, worin PyroGlu den 2-Pyrrolidon-5-carbonylrest, His den Histidylrest und ABH den 2-Aza-3-carbonyl-bicyclo[2.2.1]heptanrest bedeuten.

12. Verbindung nach den Ansprüchen 1 und 2, nämlich HomoPyroGlu-His-ABH-$NH_2$, deren Diastereoisomere, Enantiomere und Epimere sowie deren Additionssalze mit einer pharmazeutisch annehmbaren Säure, worin HomoPyroGlu den 2-Piperidinon-6-carbonylrest, His den Histidylrest und ABH den 2-Aza-3-carbonyl-bicyclo[2.2.1]heptanrest bedeuten.

13. Verbindung nach den Ansprüchen 1 und 2, nämlich HomoPyroGlu-($N^{\tau}$-Me)His-ABH-$NH_2$, deren Diastereoisomere, Enantiomere und Epimere sowie deren Additionssalze mit einer pharmazeutisch annehmbaren Säure, worin HomoPyroGlu den 2-Piperidinon-6-carbonylrest, ($N^{\tau}$-Me)His den 1-Methyl-histidylrest und ABH den 2-Aza-3-carbonyl-bicyclo[2.2.1]heptanrest bedeuten.

14. Verbindung nach den Ansprüchen 1, 3 und 5, nämlich HomoPyroGlu- Leu-MIABO-$NH_2$, deren Diastereoisomere, Enantiomere und Epimere sowie deren Additionssalze mit einer pharmazeutisch annehmbaren

Säure, worin HomoPyroGlu den 2-Piperidinon-6-carbonylrest, Leu den Leucylrest und MIABO den 4-Methyl-1-isopropyl2-aza-3-carbonyl-bicyclo[2.2.2]octanrest bedeuten.

**15.** Verbindung nach den Ansprüchen 1, 3 und 4, nämlich PyroGlu-($N^{\pi}$-Me)-His-dMABO-$NH_2$, deren Diastereoisomere, Enantiomere und Epimere sowie deren Additionssalze mit einer pharmazeutisch annehmbaren Säure, worin PyroGlu den 2-Pyrrolidinon-5-carbonylrest, ($N^{\pi}$-Me)His den 3 -Methyl-histidylrest und dMABO den 1,4-Dimethyl-2-aza-3-carbonyl-blcyclo[2.2.2]octanrest bedeuten.

**16.** Verfahren zur Herstellung der Derivate der Formel (1), **dadurch gekennzeichnet,** daß man die Aminogruppe einer Aminosäure der Formel (II), die man gegebenenfalls mit Hilfe einer klassischen Trennmethode in die Isomeren aufgetrennt hat:

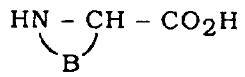 (II)

in der B die bezüglich der Formel (I) angegebenen Bedeutungen besitzt, unter der Einwirkung eines geeigneten Reagens mit einer Schutzgruppe (P), wie der tert.-Butoxycarbonylgruppe (tBOC) oder der Benzyloxycarbonylgruppe (Z) schützt, so daß man ein Derivat der Formel (III) erhält:

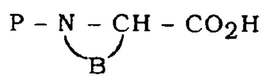 (III)

in der B und P die oben angegebenen Bedeutungen besitzen,
welches bei einer Temperatur zwischen -15 und 0°C in Gegenwart von Triethylamin mit Chlorameisensäurethylester und dann mit Ammoniak umsetzt, so daß man ein Derivat der Formel (IV) erhält:

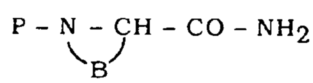 (IV)

in der B und P die oben angegebenen Bedeutungen besitzen,
von dem man mit Hilfe eines geeigneten Verfahrens, beispielsweise durch Einwirkung von gasförmiger Chlorwasserstoffsäure in einem wasserfreien Lösungsmittel, wie Dioxan oder Ethylacetat. dann, wenn P = tBOC bedeutet, oder durch katalytische Hydrierung, wenn P = Z bedeutet, die Schutzgruppe abspaltet, so daß man ein Derivat der Formel (V) erhält:

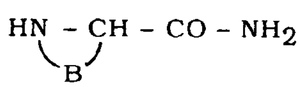 (V)

in der B die bezüglich der Formel (I) angegebenen Bedeutungen besitzt, das man gewünschtenfalls mit Hilfe klassischer Trennmethoden in die Isomeren auftrennt,
welches mit einer zweiten geschützten Aminosäure der Formel (VI):

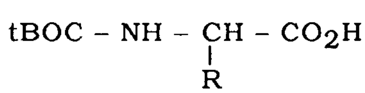 (VI)

in der R die bezüglich der Formel (I) angegebenen Bedeutungen besitzt, gekuppelt wird, so daß man ein Derivat der Formel (VII) erhält:

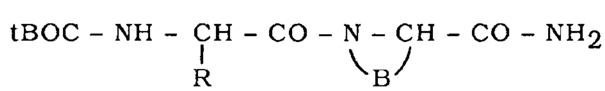 (VII)

in der R und B die bezüglich der Formel (I) angegebenen Bedeutungen besitzen, welches man gewünschtenfalls mit Hilfe einer klassischen Trennmethode in die Diastereoisomeren oder Enantiomeren trennt,
von welchen man anschließend durch Einwirkung von gasförmiger Chlorwasserstoffsäure in einem wasserfreien Lösungsmittel, wie beispielsweise Dioxan oder Ethylacetat, die Schutzgruppe abspaltet. so daß man ein Derivat der Formel (VIII) erhält:

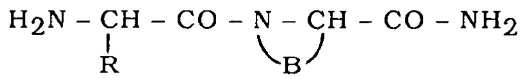

(VIII)

in der R und B die bezüglich der Formel (I) angegebenen Bedeutungen besitzen, welches mit einer dritten, gegebenenfalls geschützten Aminosäure der Formel (IX):

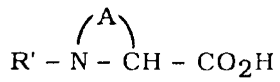

(IX)

in der R' ein Wasserstoffatom oder eine Schutzgruppe. wie beispielsweise eine Benzyloxycarbonylgruppe (Z) bedeutet, oder mit einem aktivierten Ester dieser gegebenenfalls geschützten Aminosäure gekuppelt wird, so daß man

- dann, wenn R' ein Wasserstoffatom bedeutet, ein Derivat der Formel (I) erhält, welches gewünschtenfalls mit einer pharmazeutisch annehmbaren Säure in sein Additionssalz überführt wird,
- oder wenn R' eine Schutzgruppe darstellt, ein Derivat der Formel (X) erhält:

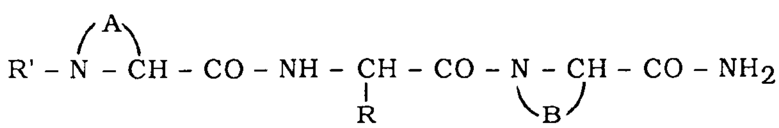

(X)

worin R und B die bezüglich der Formel (I) angegebenen Bedeutungen besitzen, von welchem man mit Hilfe einer Methode zur Abspaltung von Schutzgruppen, beispielsweise durch katalytische Hydrierung, die Schutzgruppe abspaltet. so daß man ein Derivat der Formel (I) erhält,
welches gewünschtenfalls mit einer pharmazeutisch annehmbaren Säure in sein Additionssalz umgewandelt wird, oder mit Hilfe einer klassischen Trennmethode in seine Isomeren aufgetrennt wird, die man gewünschtenfalls mit einer pharmazeutisch annehmbaren Säure in ein Salz überführt.

**17.** Verbindungen der Formel (II) nach Anspruch 16, nämlich die zur Herstellung der Verbindungen der Formel (I) geeigneten 1,4-Dialkyl-2-aza-bicyclo-[2.2.2]octan-3-carbonsäuren.

**18.** Verbindungen der Formel (III) nach Anspruch 16, die zur Herstellung der Verbindungen der Formel (I) geeignet sind.

**19.** Verbindungen der Formel (IV) nach Anspruch 16, die zur Herstellung der Verbindungen der Formel (I) geeignet sind.

**20.** Verbindungen der Formel (V) nach Anspruch 16, die zur Herstellung der Verbindungen der Formel (I) geeignet sind.

**21.** Verbindungen der Formel (VII) nach Anspruch 16. die zur Herstellung der Verbindungen der Formel (I) geeignet sind.

**22.** Verbindungen der Formel (VIII) nach Anspruch 16, die zur Herstellung der Verbindungen der Formel (I) geeignet sind.

**23.** Pharmazeutische Zubereitungen enthaltend als Wirkstoff mindestens eine Verbindung nach einem der Ansprüche 1 bis 15 allein oder in Kombination mit einem oder mehreren inerten, nichttoxischen, pharmazeutisch annehmbaren Bindemitteln oder Trägermaterialien.

**24.** Pharmazeutische Zubereitungen nach Anspruch 23, enthaltend als Wirkstoff mindestens einen Wirkstoff nach einem der Ansprüche 1 bis 15 zur Behandlung von Verhaltensstörungen und Nervenverhaltensstörungen, die mit dem Altern und akuten oder chronischen degenerativen Erkrankungen des Nervensystems verbunden sind, wie die Alzheimer'sche Krankheit, Hirngefäßtraumata, Rückgrattraumata oder amyotrophische Lateralsklerose.

**Patentansprüche für folgenden Vertragsstaat : GR**

**1.** Verfahren zur Herstellung von Verbindungen der allgemeinen Formel (I):

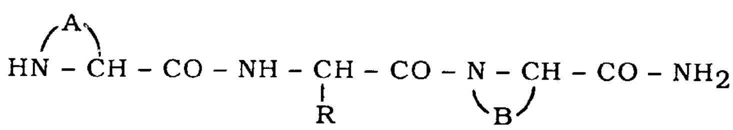
(I)

in der

A zusammen mit dem Stickstoff- und Kohlenstoffatom, an die es gebunden ist,
- eine 2-Oxo-pyrrolidin-5-yl-gruppe,
- eine 2-Oxo-piperidin-6-yl-gruppe,
- eine 2,6-Dioxo-1,2,3,6-tetrahydro-pyrimidin-4-yl-gruppe,
- eine 2-Oxo-thiazolidin-4-yl-gruppe,
- eine 2-Oxo-azetidin-4-yl-gruppe,
- eine 1-Oxo-1,2,3,4-tetrahydro-isochinol-3-yl-gruppe,

B zusammen mit dem Stickstoff- und Kohlenstoffatom, an die es gebunden ist, eine gesättigte polycyclische Struktur ausgewählt aus 2-Aza-bicyclo-[2.2.1]heptan und 1,4-(geradkettige oder verzweigte ($C_1$-$C_6$)-dialkyl)-2-aza-bicyclo[2.2.2]octanen,

R ein Wasserstoffatom, eine geradkettige oder verzweigte Niedrigalkylgruppe, eine (Imidazol-4-yl)-methylgruppe, die gegebenenfalls an einem der Stickstoffatome durch eine geradkettige oder verzweigte Niedrigalkylgruppe substituiert ist,

bedeuten, wobei der Begriff "Niedrig" darauf hinweist, daß die in dieser Weise bezeichneten Gruppen 1 bis 6 Kohlenstoffatome aufweisen, deren Enantiomeren, Diastereoisomeren und Epimeren sowie deren Additionssalzen mit einer pharmazeutisch annehmbaren Säure, **dadurch gekennzeichnet,** daß man die Aminogruppe einer Aminosäure der Formel (II), die man gegebenenfalls mit Hilfe einer klassischen Trennmethode in die Isomeren aufgetrennt hat:

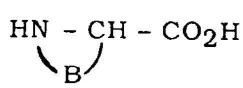
(II)

in der B die bezüglich der Formel (I) angegebenen Bedeutungen besitzt, unter der Einwirkung eines geeigneten Reagens mit einer Schutzgruppe (P), wie der tert.-Butoxycarbonylgruppe (tBOC) oder der Benzyloxycarbonylgruppe (Z) schützt, so daß man ein Derivat der Formel (III) erhält:

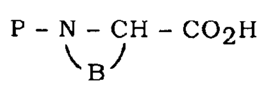
(III)

in der B und P die oben angegebenen Bedeutungen besitzen,
welches man bei einer Temperatur zwischen -15 und 0°C in Gegenwart von Triethylamin mit Chlorameisensäurethylester und dann mit Ammoniak umsetzt, so daß man ein Derivat der Formel (IV) erhält:

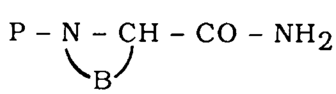
(IV)

in der B und P die oben angegebenen Bedeutungen besitzen,

von dem man mit Hilfe eines geeigneten Verfahrens, beispielsweise durch Einwirkung von gasförmiger Chlorwasserstoffsäure in einem wasserfreien Lösungsmittel, wie Dioxan oder Ethylacetat, dann, wenn P = tBOC bedeutet, oder durch katalytische Hydrierung, wenn P = Z bedeutet, die Schutzgruppe abspaltet, so daß man ein Derivat der Formel (V) erhält:

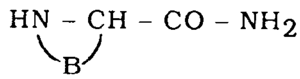

(V)

in der B die bezüglich der Formel (I) angegebenen Bedeutungen besitzt, das man gewünschtenfalls mit Hilfe klassischer Trennmethoden in die Isomeren auftrennt,
welches mit einer zweiten geschützten Aminosäure der Formel (VI):

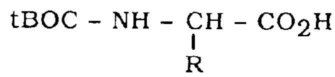

(VI)

in der R die bezüglich der Formel (I) angegebenen Bedeutungen besitzt, gekuppelt wird, so daß man ein Derivat der Formel (VII) erhält:

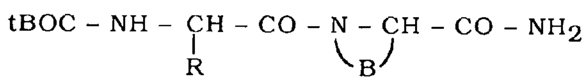

(VII)

in der R und B die bezüglich der Formel (I) angegebenen Bedeutungen besitzen, welches man gewünschtenfalls mit Hilfe einer klassischen Trennmethode in die Diastereoisomeren oder Enantiomeren trennt,
von welchen man anschließend durch Einwirkung von gasförmiger Chlorwasserstoffsäure in einem wasserfreien Lösungsmittel, wie beispielsweise Dioxan oder Ethylacetat, die Schutzgruppe abspaltet, so daß man ein Derivat der Formel (VIII) erhält:

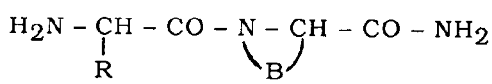

(VIII)

in der R und B die bezüglich der Formel (I) angegebenen Bedeutungen besitzen, welches mit einer dritten, gegebenenfalls geschützten Aminosäure der Formel (IX):

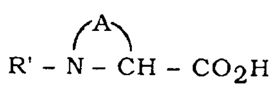

(IX)

in der R' ein Wasserstoffatom oder eine Schutzgruppe, wie beispielsweise eine Benzyloxycarbonylgruppe (Z) bedeutet,
oder mit einem aktivierten Ester dieser gegebenenfalls geschützten Aminosäure gekuppelt wird, so daß man

- dann, wenn R' ein Wasserstoffatom bedeutet, ein Derivat der Formel (I) erhält, welches gewünschtenfalls mit einer pharmazeutisch annehmbaren Säure in sein Additionssalz überführt wird,
- oder wenn R' eine Schutzgruppe darstellt, ein Derivat der Formel (X) erhält:

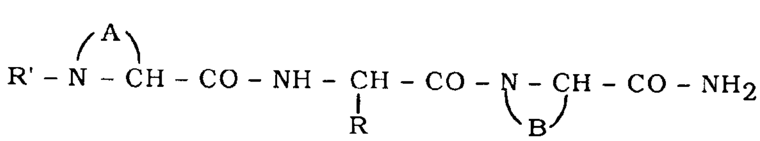

(X)

worin R und B die bezüglich der Formel (I) angegebenen Bedeutungen besitzen, von welchem man mit Hilfe einer Methode zur Abspaltung von Schutzgruppen, beispielsweise durch katalytische Hydrierung,

die Schutzgruppe abspaltet, so daß man ein Derivat der Formel (I) erhält,
welches gewünschtenfalls mit einer pharmazeutisch annehmbaren Säure in sein Additionssalz umgewandelt wird, oder mit Hilfe einer klassischen Trennmethode in seine Isomeren aufgetrennt wird, die man gewünschtenfalls mit einer pharmazeutisch annehmbaren Säure in ein Salz überführt.

2. Verfahren nach Anspruch 1 zur Herstellung von Verbindungen, worin B zusammen mit dem Stickstoff- und dem Kohlenstoffatom, an die es gebunden ist, einen 2-Aza-bicyclo[2.2.1]heptanring bildet, deren Enantiomeren, Diastereoisomeren, Epimeren sowie deren Additionssalzen mit einer pharmazeutisch annehmbaren Säure.

3. Verfahren nach Anspruch 1 zur Herstellung von Verbindungen, worin B zusammen mit dem Stickstoff- und dem Kohlenstoffatom, an die es gebunden ist, einen 1,4-Dialkyl-2-aza-bicyclo[2.2.2]octanring bildet, deren Enantiomeren, Diastereoisomeren und Epimeren sowie deren Additionssalze mit einer pharmazeutisch annehmbaren Säure.

4. Verfahren nach den Ansprüchen 1 und 3 zur Herstellung von Verbindungen, worin B zusammen mit dem Stickstoff- und dem Kohlenstoffatom, an die es gebunden ist, einen 1,4-Dimethyl-2-aza-bicyclo[2.2.2]octanring bildet, deren Enantiomeren, Diastereoisomeren und Epimeren sowie deren Additionssalze mit einer pharmazeutisch annehmbaren Säure.

5. Verfahren nach den Ansprüchen 1 und 3 zur Herstellung von Verbindungen, worin B zusammen mit dem Stickstoff- und dem Kohlenstoffatom, an die es gebunden ist, einen 4-Methyl-1-isopropyl-2-aza-bicyclo[2.2.2]octanring bildet, deren Enantiomeren, Diastereoisomeren und Epimeren sowie deren Additionssalze mit einer pharmazeutisch annehmbaren Säure.

6. Verfahren nach den Ansprüchen 1 und 2 zur Herstellung von PyroGlu- ($N^{\tau}$-Me)His-ABH-$NH_2$, deren Enantiomeren, Diastereoisomeren und Epimeren sowie deren Additionssalze mit einer pharmazeutisch annehmbaren Säure, worin PyroGlu den 2-Pyrrolidon-5-carbonylrest, ($N^{\tau}$-Me)His den 1-Methyl-histidylrest und ABH den 2-Aza-3-carbonyl-bicyclo[2.2.1]heptanrest bedeuten.

7. Verfahren nach den Ansprüchen 1 und 2 zur Herstellung von PyroGlu-Leu-ABH-$NH_2$, deren Diastereoisomeren, Enantiomeren und Epimeren sowie deren Additionssalze mit einer pharmazeutisch annehmbaren Säure, worin PyroGlu den 2-Pyrrolidon-5-carbonylrest, Leu den Leucylrest und ABH den 2-Aza-3-carbonyl-bicyclo[2.2.1]heptanrest bedeuten.

8. Verfahren nach den Ansprüchen 1 und 2 zur Herstellung von PyroGlu-($N^{\pi}$-Me)His-ABH-$NH_2$, deren Diastereoisomeren, Enantiomeren und Epimeren sowie deren Additionssalze mit einer pharmazeutisch annehmbaren Säure, worin PyroGlu den 2-Pyrrolidon-5-carbonylrest, ($N^{\pi}$-Me)His den 3-Methyl-histidylrest und ABH den 2-Aza-3-carbonyl-bicyclo[2.2.1]heptanrest bedeuten.

9. Verfahren nach den Ansprüchen 1 und 2 zur Herstellung von HomoPyro-Glu-($N^{\pi}$-Me)His-ABH-$NH_2$, deren Diastereoisomeren, Enantiomeren und Epimeren sowie deren Additionssalze mit einer pharmazeutisch annehmbaren Säure, worin HomoPyroGlu den 2-Piperidinon-6-carbonylrest, ($N^{\pi}$-Me)His den 3-Methyl-histidylrest und ABH den 2-Aza-3-carbonyl-bicyclo[2.2.1]heptanrest bedeuten.

10. Verfahren nach den Ansprüchen 1 und 2 zur Herstellung von HomoPyro-Glu-Leu-ABH-$NH_2$, deren Diastereoisomeren, Enantiomeren und Epimeren sowie deren Additionssalze mit einer pharmazeutisch annehmbaren Säure, worin HomoPyroGlu den 2-Piperidinon-6-carbonylrest, Leu den Leucylrest und ABH den 2-Aza-3-carbonyl-bicyclo[2.2.1]heptanrest bedeuten.

11. Verfahren nach den Ansprüchen 1 und 2 zur Herstellung von PyroGluHis-ABH-$NH_2$, deren Diastereoisomeren, Enantiomeren und Epimeren sowie deren Additionssalze mit einer pharmazeutisch annehmbaren Säure, worin PyroGlu den 2-Pyrrolidon-5-carbonylrest. His den Histidylrest und ABH den 2-Aza-3-carbonyl-bicyclo[2.2.1]heptanrest bedeuten.

12. Verfahren nach den Ansprüchen 1 und 2 zur Herstellung von HomoPyro-Glu-His-ABH-$NH_2$, deren Diastereoisomeren, Enantiomeren und Epimeren sowie deren Additionssalze mit einer pharmazeutisch annehmbaren Säure, worin HomoPyroGlu den 2-Piperidinon-6-carbonylrest, His den Histidylrest und ABH den 2-Aza-3-carbonyl-bicyclo[2.2.1]heptanrest bedeuten.

**13.** Verfahren nach den Ansprüchen 1 und 2 zur Herstellung von HomoPyro-Glu-($N^{\tau}$-Me)His-ABH-$NH_2$, deren Diastereoisomeren, Enantiomeren und Epimeren sowie deren Additionssalze mit einer pharmazeutisch annehmbaren Säure, worin HomoPyroGlu den 2-Piperidinon-6-carbonylrest, ($N^{\tau}$-Me)His den 1-Methyl-histidylrest und ABH den 2-Aza-3-carbonyl-bicyclo[2.2.1]heptanrest bedeuten.

**14.** Verfahren nach den Ansprüchen 1, 3 und 5 zur Herstellung von HomoPyroGlu-Leu-MIABO-$NH_2$, deren Diastereoisomeren, Enantiomeren und Epimeren sowie deren Additionssalze mit einer pharmazeutisch annehmbaren Säure, worin HomoPyroGlu den 2-Piperidinon-6-carbonylrest, Leu den Leucylrest und MIABO den 4-Methyl- 1 -isopropyl2-aza-3-carbonyl-bicyclo[2.2.2]octanrest bedeuten.

**15.** Verfahren nach den Ansprüchen 1, 3 und 4 zur Herstellung von PyroGlu-($N^{\pi}$-Me)His-dMABO-$NH_2$. deren Diastereoisomeren, Enantiomeren und Epimeren sowie deren Additionssalze mit einer pharmazeutisch annehmbaren Säure, worin PyroGlu den 2-Pyrrolidinon-5-carbonylrest, ($N^{\pi}$-Me)His den 3-Methyl-histidylrest und dMABO den 1.4-Dimethyl-2-aza-3-carbonyl-bicyclo[2.2.2]octanrest bedeuten.

**16.** Verfahren nach Anspruch 1 zur Herstellung von Verbindungen der Formel (II), die zur Herstellung der Verbindungen der Formel (I) geeignet sind.

**17.** Verfahren nach Anspruch 1 zur Herstellung von Verbindungen der Formel (III), die zur Herstellung der Verbindungen der Formel (I) geeignet sind.

**18.** Verfahren nach Anspruch 1 zur Herstellung von Verbindungen der Formel (IV), die zur Herstellung der Verbindungen der Formel (I) geeignet sind.

**19.** Verfahren nach Anspruch 1 zur Herstellung von Verbindungen der Formel (V), die zur Herstellung der Verbindungen der Formel (I) geeignet sind.

**20.** Verfahren nach Anspruch 1 zur Herstellung von Verbindungen der Formel (VII), die zur Herstellung der Verbindungen der Formel (I) geeignet sind.

**21.** Verfahren nach Anspruch 1 zur Herstellung von Verbindungen der Formel (VIII), die zur Herstellung der Verbindungen der Formel (I) geeignet sind.

**Patentansprüche für folgenden Vertragsstaat : ES**

**1.** Verfahren zur Herstellung von Verbindungen der allgemeinen Formel (I):

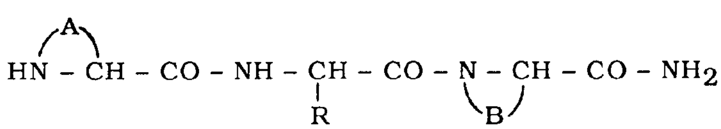

(I)

in der

A zusammen mit dem Stickstoff- und Kohlenstoffatom, an die es gebunden ist,
- eine 2-Oxo-pyrrolidin-5-yl-gruppe,
- eine 2-Oxo-piperidin-6-yl-gruppe,
- eine 2,6-Dioxo-1,2,3,6-tetrahydro-pyrimidin-4-yl-gruppe,
- eine 2-Oxo-thiazolidin-4-yl-gruppe,
- eine 2-Oxo-azetidin-4-yl-gruppe,
- eine 1-Oxo-1,2,3,4-tetrahydro-isochinol-3-yl-gruppe,

B zusammen mit dem Stickstoff- und Kohlenstoffatom, an die es gebunden ist, eine gesättigte polycyclische Struktur ausgewählt aus 2-Aza-bicyclo-[2.2.1]heptan und 1,4-(geradkettige oder verzweigte ($C_1$-$C_6$)-dialkyl)-2-aza-bicyclo[2.2.2]octanen,

R ein Wasserstoffatom, eine geradkettige oder verzweigte Niedrigalkylgruppe, eine (Imidazol-4-yl)-methylgruppe, die gegebenenfalls an einem der Stickstoffatome durch eine geradkettige oder verzweigte Niedrigalkylgruppe substituiert ist,

bedeuten, wobei der Begriff "Niedrig" darauf hinweist, daß die in dieser Weise bezeichneten Gruppen 1 bis 6 Kohlenstoffatome aufweisen, deren Enantiomeren, Diastereoisomeren und Epimeren sowie deren

Additionssalzen mit einer pharmazeutisch annehmbaren Säure, **dadurch gekennzeichnet**, daß man die Aminogruppe einer Aminosäure der Formel (II), die man gegebenenfalls mit Hilfe einer klassischen Trennmethode in die Isomeren aufgetrennt hat:

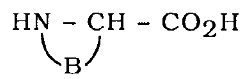 (II)

in der B die bezüglich der Formel (I) angegebenen Bedeutungen besitzt, unter der Einwirkung eines geeigneten Reagens mit einer Schutzgruppe (P), wie der tert.-Butoxycarbonylgruppe (tBOC) oder der Benzyloxycarbonylgruppe (Z) schützt, so daß man ein Derivat der Formel (III) erhält:

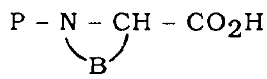 (III)

in der B und P die oben angegebenen Bedeutungen besitzen,
welches man bei einer Temperatur zwischen -15 und 0°C in Gegenwart von Triethylamin mit Chlorameisensäurethylester und dann mit Ammoniak umsetzt, so daß man ein Derivat der Formel (IV) erhält:

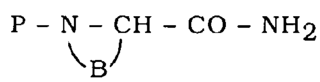 (IV)

in der B und P die oben angegebenen Bedeutungen besitzen,
von dem man mit Hilfe eines geeigneten Verfahrens, beispielsweise durch Einwirkung von gasförmiger Chlorwasserstoffsäure in einem wasserfreien Lösungsmittel, wie Dioxan oder Ethylacetat, dann, wenn P = tBOC bedeutet, oder durch katalytische Hydrierung, wenn P = Z bedeutet, die Schutzgruppe abspaltet, so daß man ein Derivat der Formel (V) erhält:

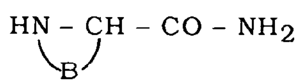 (V)

in der B die bezüglich der Formel (I) angegebenen Bedeutungen besitzt,
das man gewünschtenfalls mit Hilfe klassischer Trennmethoden in die Isomeren auftrennt,
welches mit einer zweiten geschützten Aminosäure der Formel (VI):

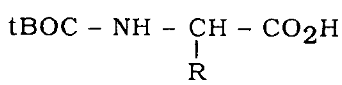 (VI)

in der R die bezüglich der Formel (I) angegebenen Bedeutungen besitzt, gekuppelt wird, so daß man ein Derivat der Formel (VII) erhält:

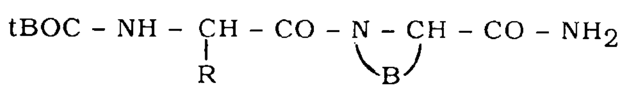 (VII)

in der R und B die bezüglich der Formel (I) angegebenen Bedeutungen besitzen, welches man gewünschtenfalls mit Hilfe einer klassischen Trennmethode in die Diastereoisomeren oder Enantiomeren trennt,
von welchen man anschließend durch Einwirkung von gasförmiger Chlorwasserstoffsäure in einem wasserfreien Lösungsmittel, wie beispielsweise Dioxan oder Ethylacetat, die Schutzgruppe abspaltet, so daß man ein Derivat der Formel (VIII) erhält:

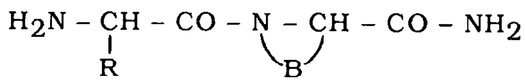 (VIII)

in der R und B die bezüglich der Formel (I) angegebenen Bedeutungen besitzen, welches mit einer dritten, gegebenenfalls geschützten Aminosäure der Formel (IX):

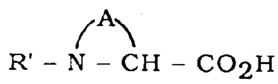 (IX)

in der R' ein Wasserstoffatom oder eine Schutzgruppe, wie beispielsweise eine Benzyloxycarbonylgruppe (Z) bedeutet,
oder mit einem aktivierten Ester dieser gegebenenfalls geschützten Aminosäure gekuppelt wird, so daß man

- dann, wenn R' ein Wasserstoffatom bedeutet, ein Derivat der Formel (I) erhält, welches gewünschtenfalls mit einer pharmazeutisch annehmbaren Säure in sein Additionssalz überführt wird,
- oder wenn R' eine Schutzgruppe darstellt, ein Derivat der Formel (X) erhält:

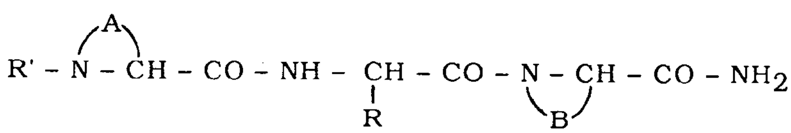 (X)

worin R und B die bezüglich der Formel (I) angegebenen Bedeutungen besitzen, von welchem man mit Hilfe einer Methode zur Abspaltung von Schutzgruppen, beispielsweise durch katalytische Hydrierung, die Schutzgruppe abspaltet, so daß man ein Derivat der Formel (I) erhält,
welches gewünschtenfalls mit einer pharmazeutisch annehmbaren Säure in sein Additionssalz umgewandelt wird, oder mit Hilfe einer klassischen Trennmethode in seine Isomeren aufgetrennt wird, die man gewünschtenfalls mit einer pharmazeutisch annehmbaren Säure in ein Salz überführt.

2. Verfahren nach Anspruch 1 zur Herstellung von Verbindungen, worin B zusammen mit dem Stickstoff- und dem Kohlenstoffatom, an die es gebunden ist, einen 2-Aza-bicyclo[2.2.1]heptanring bildet, deren Enantiomeren, Diastereoisomeren, Epimeren sowie deren Additionssalzen mit einer pharmazeutisch annehmbaren Säure.

3. Verfahren nach Anspruch 1 zur Herstellung von Verbindungen, worin B zusammen mit dem Stickstoff- und dem Kohlenstoffatom, an die es gebunden ist, einen 1,4-Dialkyl-2-aza-bicyclo[2.2.2]octanring bildet, deren Enantiomeren, Diastereoisomeren und Epimeren sowie deren Additionssalze mit einer pharmazeutisch annehmbaren Säure.

4. Verfahren nach den Ansprüchen 1 und 3 zur Herstellung von Verbindungen, worin B zusammen mit dem Stickstoff- und dem Kohlenstoffatom, an die es gebunden ist, einen 1,4-Dimethyl-2-aza-bicyclo[2.2.2]octanring bildet, deren Enantiomeren, Diastereoisomeren und Epimeren sowie deren Additionssalze mit einer pharmazeutisch annehmbaren Säure.

5. Verfahren nach den Ansprüchen 1 und 3 zur Herstellung von Verbindungen, worin B zusammen mit dem Stickstoff- und dem Kohlenstoffatom, an die es gebunden ist, einen 4-Methyl-1-isopropyl-2-aza-bicyclo[2.2.2]octanring bildet, deren Enantiomeren, Diastereoisomeren und Epimeren sowie deren Additionssalze mit einer pharmazeutisch annehmbaren Säure.

6. Verfahren nach den Ansprüchen 1 und 2 zur Herstellung von PyroGlu-($N^{\tau}$-Me)His-ABH-$NH_2$, deren Enantiomeren, Diastereoisomeren und Epimeren sowie deren Additionssalze mit einer pharmazeutisch annehmbaren Säure, worin PyroGlu den 2-Pyrrolidon-5-carbonylrest, ($N^{\tau}$-Me)His den 1-Methyl-histidylrest und ABH den 2-Aza-3-carbonyl-bicyclo[2.2.1]heptanrest bedeuten.

7. Verfahren nach den Ansprüchen 1 und 2 zur Herstellung von PyroGlu-Leu-ABH-$NH_2$, deren Diastereoisomeren, Enantiomeren und Epimeren sowie deren Additionssalze mit einer pharmazeutisch annehmbaren Säure

baren Säure, worin PyroGlu den 2-Pyrrolidon-5-carbonylrest, Leu den Leucylrest und ABH den 2-Aza-3-carbonyl-bicyclo[2.2.1]heptanrest bedeuten.

8. Verfahren nach den Ansprüchen 1 und 2 zur Herstellung von PyroGlu-($N^{\pi}$-Me)His-ABH-$NH_2$, deren Diastereoisomeren, Enantiomeren und Epimeren sowie deren Additionssalze mit einer pharmazeutisch annehmbaren Säure, worin PyroGlu den 2-Pyrrolldon-5-carbonylrest, ($N^{\pi}$-Me)His den 3-Methyl-histidylrest und ABH den 2-Aza-3-carbonyl-bicyclo[2.2.1]heptanrest bedeuten.

9. Verfahren nach den Ansprüchen 1 und 2 zur Herstellung von HomoPyro-Glu-($N^{\pi}$-Me)His-ABH-$NH_2$. deren Diastereoisomeren, Enantiomeren und Epimeren sowie deren Additionssalze mit einer pharmazeutisch annehmbaren Säure, worin HomoPyroGlu den 2-Piperidinon-6-carbonylrest, ($N^{\pi}$-Me)His den 3-Methyl-histidylrest und ABH den 2-Aza-3-carbonyl-bicyclo[2.2.1]heptanrest bedeuten.

10. Verfahren nach den Ansprüchen 1 und 2 zur Herstellung von HomoPyro-Glu-Leu-ABH-$NH_2$, deren Diastereoisomeren, Enantiomeren und Epimeren sowie deren Additionssalze mit einer pharmazeutisch annehmbaren Säure, worin HomoPyroGlu den 2-Piperidinon-6-carbonylrest, Leu den Leucylrest und ABH den 2-Aza-3-carbonyl-bicyclo[2.2.1]heptanrest bedeuten.

11. Verfahren nach den Ansprüchen 1 und 2 zur Herstellung von PyroGlu-His-ABH-$NH_2$, deren Diastereoisomeren, Enantiomeren und Epimeren sowie deren Additionssalze mit einer pharmazeutisch annehmbaren Säure, worin PyroGlu den 2-Pyrrolidon-5-carbonylrest, His den Histidylrest und ABH den 2-Aza-3-carbonyl-bicyclo[2.2.1]heptanrest bedeuten.

12. Verfahren nach den Ansprüchen 1 und 2 zur Herstellung von HomoPyro-Glu-His-ABH-$NH_2$, deren Diastereoisomeren, Enantiomeren und Epimeren sowie deren Additionssalze mit einer pharmazeutisch annehmbaren Säure, worin HomoPyroGlu den 2-Piperidinon-6-carbonylrest, His den Histidylrest und ABH den 2-Aza-3-carbonyl-bicyclo[2.2.1]heptanrest bedeuten.

13. Verfahren nach den Ansprüchen 1 und 2 zur Herstellung von HomoPyro-Glu-($N^{\tau}$-Me)His-ABH-$NH_2$, deren Diastereoisomeren, Enantiomeren und Epimeren sowie deren Additionssalze mit einer pharmazeutisch annehmbaren Säure, worin HomoPyroGlu den 2-Piperidinon-6-carbonylrest, ($N^{\tau}$-Me)His den 1-Methyl-histidylrest und ABH den 2-Aza-3-carbonyl-bicyclo[2.2.1]heptanrest bedeuten.

14. Verfahren nach den Ansprüchen 1, 3 und 5 zur Herstellung von HomoPyroGlu-Leu-MIABO-$NH_2$, deren Diastereoisomeren, Enantiomeren und Epimeren sowie deren Additionssalze mit einer pharmazeutisch annehmbaren Säure, worin HomoPyroGlu den 2-Piperidinon-6-carbonylrest, Leu den Leucylrest und MIABO den 4-Methyl-1-isopropyl2-aza-3-carbonyl-bicyclo[2.2.2]octanrest bedeuten.

15. Verfahren nach den Ansprüchen 1, 3 und 4 zur Herstellung von PyroGlu-($N^{\pi}$- Me)His-dMABO-$NH_2$, deren Diastereoisomeren, Enantiomeren und Epimeren sowie deren Additionssalze mit einer pharmazeutisch annehmbaren Säure, worin PyroGlu den 2-Pyrrolidinon-5-carbonylrest, ($N^{\pi}$-Me)His den 3-Methyl-histidylrest und dMABO den 1,4-Dimethyl-2-aza-3-carbonyl-bicyclo[2.2.2]octanrest bedeuten.

16. Verfahren nach Anspruch 1 zur Herstellung von Verbindungen der Formel (II), die zur Herstellung der Verbindungen der Formel (I) geeignet sind.

17. Verfahren nach Anspruch 1 zur Herstellung von Verbindungen der Formel (III), die zur Herstellung der Verbindungen der Formel (I) geeignet sind.

18. Verfahren nach Anspruch 1 zur Herstellung von Verbindungen der Formel (IV), die zur Herstellung der Verbindungen der Formel (I) geeignet sind.

19. Verfahren nach Anspruch 1 zur Herstellung von Verbindungen der Formel (V), die zur Herstellung der Verbindungen der Formel (I) geeignet sind.

20. Verfahren nach Anspruch 1 zur Herstellung von Verbindungen der Formel (VII), die zur Herstellung der Verbindungen der Formel (I) geeignet sind.

21. Verfahren nach Anspruch 1 zur Herstellung von Verbindungen der Formel (VIII), die zur Herstellung der Verbindungen der Formel (I) geeignet sind.

## Claims

### Claims for the following Contracting States : AT, BE, CH, DE, DK, FR, GB, IT, LI, LU, NL, SE

1. Compounds of the general formula (I):

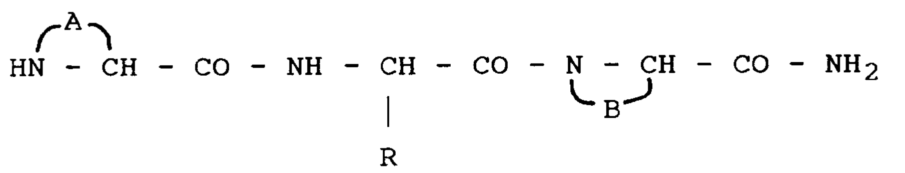

(I)

in which:

A represents, together with the nitrogen and carbon atoms to which it is bonded:
- a 2-oxopyrrolidin-5-yl grouping,
- a 2-oxopiperidin-6-yl grouping,
- a 2,6-dioxo-1,2,3,6-tetrahydropyrimidin-4-yl grouping,
- a 2-oxothiazolidin-4-yl grouping,
- a 2-oxoazetidin-4-yl grouping, or
- a 1-oxo-1,2,3,4-tetrahydroisoquinol-3-yl grouping,

B represents, together with the nitrogen and carbon atoms to which it is bonded, a saturated polycyclic structure selected from 2-azabicyclo-[2.2.1]-heptane and the 1,4-di-(linear and branched $(C_1-C_6)$alkyl)-2-azabicyclo[2.2.2]-octanes, and

R represents a hydrogen atom, a linear or branched lower alkyl grouping, or a 4-imidazolylmethyl grouping optionally substituted at one of the nitrogen atoms by a linear or branched lower alkyl radical, the term "lower" indicating that the groupings so described have from 1 to 6 carbon atoms,

their enantiomers, diastereoisomers and epimers and also their addition salts with a pharmaceutically acceptable acid.

2. Compounds according to claim 1 such that B forms, together with the nitrogen and carbon atoms to which it is bonded, a 2-azabicyclo[2.2.1]heptane ring, their enantiomers, diastereoisomers and epimers and also their addition salts with a pharmaceutically acceptable acid.

3. Compounds according to claim 1 such that B forms, together with the nitrogen and carbon atoms to which it is bonded, a 1,4-dialkyl-2-azabicyclo[2.2.2]octane ring, their enantiomers, diastereoisomers and epimers and also their addition salts with a pharmaceutically acceptable acid.

4. Compounds according to claims 1 and 3 such that B forms, together with the nitrogen and carbon atoms to which it is bonded, a 1,4-dimethyl-2-azabicyclo[2.2.2]-octane ring, their enantiomers, diastereoisomers and epimers and also their addition salts with a pharmaceutically acceptable acid.

5. Compounds according to claims 1 and 3 such that B forms, together with the nitrogen and carbon atoms to which it is bonded, a 4-methyl-1-isopropyl-2-azabicyclo-[2.2.2]octane ring, their enantiomers, diastereoisomers and epimers and also their addition salts with a pharmaceutically acceptable acid.

6. Compound according to claims 1 and 2 which is PyroGlu-($N^{\tau}$-Me)His-ABH-$NH_2$, its enantiomers, diastereoisomers and epimers, and also its addition salts with a pharmaceutically acceptable acid, PyroGlu representing the radical 2-pyrrolidone-5-carbonyl, ($N^{\tau}$-Me)His representing the radical 1-methylhistidyl and ABH the radical 2-aza-3-carbonylbicyclo[2.2.1]heptane.

7. Compound according to claims 1 and 2 which is PyroGlu-Leu-ABH-$NH_2$, its diastereoisomers, enantiomers and epimers, and also its addition salts with a pharmaceutically acceptable acid, PyroGlu representing the radical 2-pyrrolidone-5-carbonyl, Leu representing the radical leucyl and ABH the radical 2-aza-3-carbonylbicyclo[2.2.1]heptane.

8. Compound according to claims 1 and 2 which is PyroGlu-($N^{\pi}$-Me)His-ABH-$NH_2$, its diastereoisomers, enantiomers and epimers, and also its addition salts with a pharmaceutically acceptable acid, PyroGlu representing the radical 2-pyrrolidone-5-carbonyl, ($N^{\pi}$-Me)His the radical 3-methylhistidyl and ABH the radical 2-aza-3-carbonylbicyclo[2.2.1]heptane.

**9.** Compound according to claims 1 and 2 which is HomoPyroGlu-($N^{\pi}$-Me)His-ABH-$NH_2$, its diastereoisomers, enantiomers and epimers, and also its addition salts with a pharmaceutically acceptable acid, HomoPyroGlu representing the radical 2-piperidinone-6-carbonyl, ($N^{\pi}$-Me)His the radical 3-methylhistidyl and ABH the radical 2-aza-3-carbonylbicyclo[2.2.1]heptane.

**10.** Compound according to claims 1 and 2 which is HomoPyro-Glu-Leu-ABH-$NH_2$, its diastereoisomers, enantiomers and epimers, and also its addition salts with a pharmaceutically acceptable acid, HomoPyroGlu representing the radical 2-piperidinone-6-carbonyl, Leu representing the radical leucyl and ABH the radical 2-aza-3-carbonylbicyclo[2.2.1]heptane.

**11.** Compound according to claims 1 and 2 which is PyroGlu-His-ABH-$NH_2$, its diastereoisomers, enantiomers and epimers, and also its addition salts with a pharmaceutically acceptable acid, PyroGlu representing the radical 2-pyrrolidone-5-carbonyl, His representing the radical histidyl and ABH the radical 2-aza-3-carbonylbicyclo[2.2.1]heptane.

**12.** Compound according to claims 1 and 2 which is HomoPyroGlu-His-ABH-$NH_2$, its diastereoisomers, enantiomers and epimers, and also its addition salts with a pharmaceutically acceptable acid, HomoPyroGlu representing the radical 2-piperidinone-6-carbonyl, His the radical histidyl and ABH the radical 2-aza-3-carbonylbicyclo[2.2.1]heptane.

**13.** Compound according to claims 1 and 2 which is HomoPyroGlu-($N^{\tau}$-Me)His-ABH-$NH_2$, its diastereoisomers, enantiomers and epimers, and also its addition salts with a pharmaceutically acceptable acid, HomoPyroGlu representing the radical 2-piperidinone-6-carbonyl, ($N^{\tau}$-Me)His the radical 1-methylhistidyl and ABH the radical 2-aza-3-carbonylbicyclo[2.2.1]heptane.

**14.** Compound according to claims 1, 3 and 5 which is HomoPyroGlu-Leu-MIABO-$NH_2$, its diastereoisomers, enantiomers and epimers, and also its addition salts with a pharmaceutically acceptable acid, HomoPyroGlu representing the radical 2-piperidinone-6-carbonyl, Leu representing the radical leucyl and MIABO the radical 4-methyl-1-isopropyl-2-aza-3-carbonylbicyclo[2.2.2]-octane.

**15.** Compound according to claims 1, 3 and 4 which is PyroGlu-($N^{\pi}$-Me)His-dMABO-$NH_2$, its diastereoisomers, enantiomers and epimers, and also its addition salts with a pharmaceutically acceptable acid, PyroGlu representing the radical 2-pyrrolidinone-5-carbonyl, ($N^{\pi}$-Me)His the radical 3-methylhistidyl and dMABO the radical 1,4-dimethyl-2-aza-3-carbonylbicyclo[2.2.2]octane.

**16.** Process for the preparation of the compounds of formula (I), characterised in that the amine function of an amino acid of formula (II):

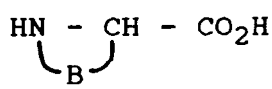

(II)

in which B has the same meaning as in formula (I),
the isomers of which have optionally been separated by a conventional separation technique, is protected by a protecting radical (P), such as tert.-butoxycarbonyl (tBOC) or benzyloxycarbonyl (Z), under the action of a suitable reagent to give a compound of formula (III):

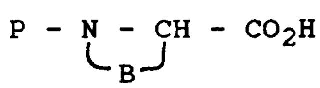

(III)

in which B and P have the same meanings as given above,
on which ethyl chloroformate and then ammonium hydroxide are caused to react, at a temperature of between -15 and 0°C, in the presence of triethylamine,
to give a compound of formula (IV):

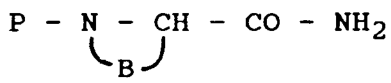 (IV)

in which B and P have the same meanings as given above,
which is deprotected by a suitable process, such as, for example, by the action of gaseous HCl in an anhydrous solvent, such as dioxane or ethyl acetate, when P = tBOC or by catalytic hydrogenation when P = Z,
to give a compound of formula (V):

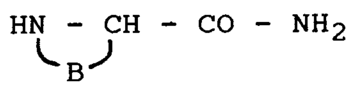 (V)

in which B has the same meaning as in formula (I),
the isomers of which are, if desired, separated by a conventional separation technique,
which is coupled with a second protected amino acid of formula (VI):

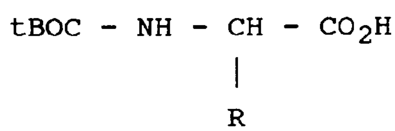 (VI)

in which R has the same meaning as in formula (I),
to give a compound of formula (VII):

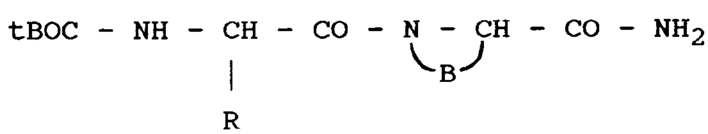 (VII)

in which R and B have the same meanings as in formula (I), the diastereoisomers or enantiomers of which are, if desired, separated by a conventional separation technique,
which is then deprotected by the action of gaseous HCl in an anhydrous solvent, such as, for example, dioxane or ethyl acetate, to give a compound of formula (VIII):

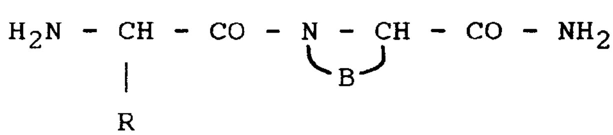 (VIII)

in which R and B have the same meanings as in formula (I),
which is coupled with a third optionally protected amino acid of formula (IX):

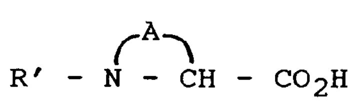 (IX)

in which R' is a hydrogen atom, or a protecting grouping, such as, for example, benzyloxycarbonyl (Z), or with an activated ester of that optionally protected amino acid, to give:

- either a compound of formula (I) when R' is a hydrogen atom, which is, if desired, converted into its addition salt with a pharmaceutically acceptable acid,
- or a compound of formula (X):

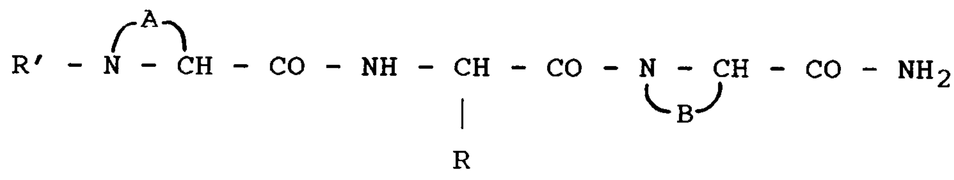 (X)

when R' is a protecting grouping, R and B having the same meanings as in formula (I), which is deprotected by a deprotection technique, such as, for example, catalytic hydrogenation, to give a compound of formula (I), which is, if desired, converted into its addition salt with a pharmaceutically acceptable acid or separated into its isomers according to a conventional separation technique, which isomers are, if desired, converted into their salts by means of a pharmaceutically acceptable acid.

**17.** Compounds of formula (II) according to claim 16 which are 1,4-dialkyl-2-azabicyclo[2.2.2]octane-3-carboxylic acids, which can be used in the preparation of compounds of formula (I).

**18.** Compounds of formula (III) according to claim 16 which can be used in the preparation of compounds of formula (I).

**19.** Compounds of formula (IV) according to claim 16 which can be used in the preparation of compounds of formula (I).

**20.** Compounds of formula (V) according to claim 16 which can be used in the preparation of compounds of formula (I).

**21.** Compounds of formula (VII) according to claim 16 which can be used in the preparation of compounds of formula (I).

**22.** Compounds of formula (VIII) according to claim 16 which can be used in the preparation of compounds of formula (I).

**23.** Pharmaceutical compositions containing as active ingredient at least one compound according to any one of claims 1 to 15, alone or in combination with one or more inert non-toxic pharmaceutically acceptable excipients or carriers.

**24.** Pharmaceutical compositions according to claim 23 containing at least one active ingredient according to one of claims 1 to 15 which can be used in the treatment of constitutive disorders and neurobehavioural disorders associated with ageing and acute or chronic degenerative diseases of the nervous system, such as Alzheimer's disease, cerebral vascular accident, spinal traumatism or lateral amyotrophic sclerosis.

**Claims for the following Contracting State : GR**

**1.** Process for the preparation of compounds of the general formula (I):

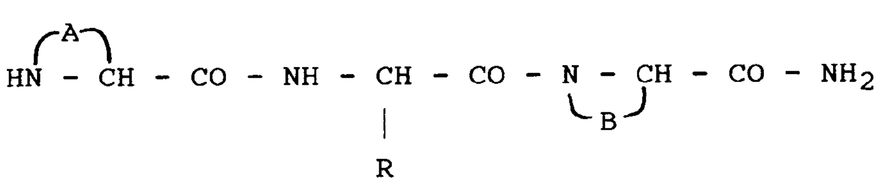 (I)

in which:

A represents, together with the nitrogen and carbon atoms to which it is bonded:

- a 2-oxopyrrolidin-5-yl grouping,
- a 2-oxopiperidin-6-yl grouping,
- a 2,6-dioxo-1,2,3,6-tetrahydropyrimidin-4-yl grouping,
- a 2-oxothiazolidin-4-yl grouping,

- a 2-oxoazetidin-4-yl grouping, or
- a 1-oxo-1,2,3,4-tetrahydroisoquinol-3-yl grouping,

B represents, together with the nitrogen and carbon atoms to which it is bonded, a saturated polycyclic structure selected from 2-azabicyclo-[2.2.1]-heptane and the 1,4-di-(linear and branched ($C_1$-$C_6$)alkyl)-2-azabicyclo[2.2.2]-octanes, and

R represents a hydrogen atom, a linear or branched lower alkyl grouping, or a 4-imidazolylmethyl grouping optionally substituted at one of the nitrogen atoms by a linear or branched lower alkyl radical, the term "lower" indicating that the groupings so described have from 1 to 6 carbon atoms,

their enantiomers, diastereoisomers and epimers and also their addition salts with a pharmaceutically acceptable acid, characterised in that the amine function of an amino acid of formula (II):

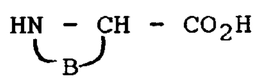 (II)

in which B has the same meaning as in formula (I),
the isomers of which have optionally been separated by a conventional separation technique, is protected by a protecting radical (P), such as tert.-butoxycarbonyl (tBOC) or benzyloxycarbonyl (Z), under the action of a suitable reagent to give a compound of formula (III):

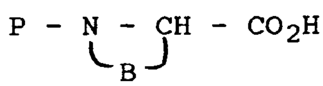 (III)

in which B and P have the same meanings as given above,
on which ethyl chloroformate and then ammonium hydroxide are caused to react, at a temperature of between -15 and 0°C, in the presence of triethylamine,
to give a compound of formula (IV):

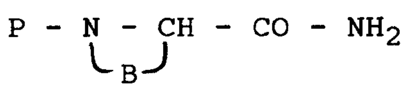 (IV)

in which B and P have the same meanings as given above,
which is deprotected by a suitable process, such as, for example, by the action of gaseous HCl in an anhydrous solvent, such as dioxane or ethyl acetate, when P = tBOC or by catalytic hydrogenation when P = Z,
to give a compound of formula (V):

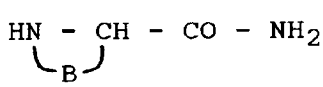 (V)

in which B has the same meaning as in formula (I),
the isomers of which are, if desired, separated by a conventional separation technique,
which is coupled with a second protected amino acid of formula (VI):

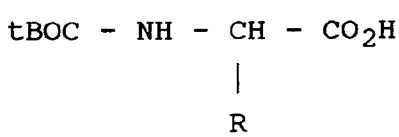 (VI)

in which R has the same meaning as in formula (I),
to give a compound of formula (VII):

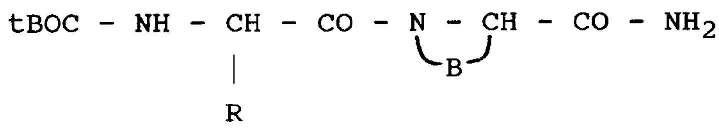

(VII)

in which R and B have the same meanings as in formula (I), the diastereoisomers or enantiomers of which are, if desired, separated by a conventional separation technique,
which is then deprotected by the action of gaseous HCl in an anhydrous solvent, such as, for example, dioxane or ethyl acetate, to give a compound of formula (VIII):

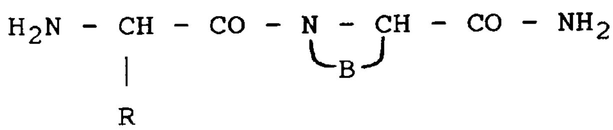

(VIII)

in which R and B have the same meanings as in formula (I),
which is coupled with a third optionally protected amino acid of formula (IX):

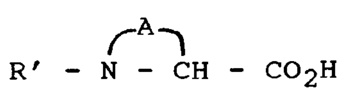

(IX)

in which R' is a hydrogen atom, or a protecting grouping, such as, for example, benzyloxycarbonyl (Z), or with an activated ester of that optionally protected amino acid, to give:

- either a compound of formula (I) when R' is a hydrogen atom, which is, if desired, converted into its addition salt with a pharmaceutically acceptable acid,
- or a compound of formula (X):

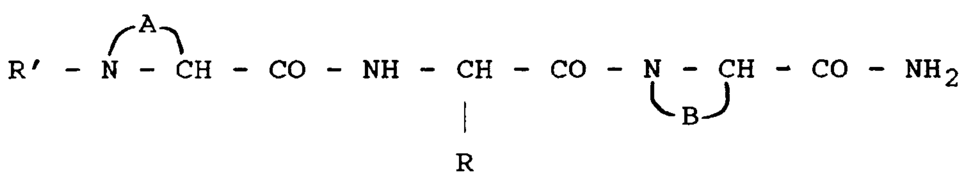

(X)

when R' is a protecting grouping, R and B having the same meanings as in formula (I), which is deprotected by a deprotection technique, such as, for example, catalytic hydrogenation, to give a compound of formula (I),

which is, if desired, converted into its addition salt with a pharmaceutically acceptable acid or separated into its isomers according to a conventional separation technique, which isomers are, if desired, converted into their salts by means of a pharmaceutically acceptable acid.

2. Process according to claim 1 for the preparation of compounds such that B forms, together with the nitrogen and carbon atoms to which it is bonded, a 2-azabicyclo-[2.2.1]heptane ring, their enantiomers, diastereoisomers and epimers and also their addition salts with a pharmaceutically acceptable acid.

3. Process according to claim 1 for the preparation of compounds such that B forms, together with the nitrogen and carbon atoms to which it is bonded, a 1,4-dialkyl-2-azabicyclo[2.2.2]octane ring, their enantiomers, diastereoisomers and epimers and also their addition salts with a pharmaceutically acceptable acid.

4. Process according to claims 1 and 3 for the preparation of compounds such that B forms, together with the nitrogen and carbon atoms to which it is bonded, a 1,4-dimethyl-2-azabicyclo[2.2.2]octane ring, their enantiomers, diastereoisomers and epimers and also their addition salts with a pharmaceutically acceptable acid.

5. Process according to claims 1 and 3 for the preparation of compounds such that B forms, together with the nitrogen and carbon atoms to which it is bonded, a 4-methyl-1-isopropyl-2-azabicyclo[2.2.2]octane ring, their enantiomers, diastereoisomers and epimers and also their addition salts with a pharmaceutically acceptable acid.

6. Process according to claims 1 and 2 for the preparation of the compound PyroGlu-($N^{\tau}$-Me)His-ABH-$NH_2$, its enantiomers, diastereoisomers and epimers, and also its addition salts with a pharmaceutically acceptable acid, PyroGlu representing the radical 2-pyrrolidone-5-carbonyl, ($N^{\tau}$-Me)His representing the radical 1-methylhistidyl and ABH the radical 2-aza-3-carbonylbicyclo-[2.2.1]heptane.

7. Process according to claims 1 and 2 for the preparation of the compound PyroGlu-Leu-ABH-$NH_2$, its diastereoisomers, enantiomers and epimers, and also its addition salts with a pharmaceutically acceptable acid, PyroGlu representing the radical 2-pyrrolidone-5-carbonyl, Leu representing the radical leucyl and ABH the radical 2-aza-3-carbonylbicyclo[2.2.1]heptane.

8. Process according to claims 1 and 2 for the preparation of the compound PyroClu-($N^{\pi}$-Me)His-ABH-$NH_2$, its diastereoisomers, enantiomers and epimers, and also its addition salts with a pharmaceutically acceptable acid, PyroGlu representing the radical 2-pyrrolidone-5-carbonyl, ($N^{\pi}$-Me)His the radical 3-methylhistidyl and ABH the radical 2-aza-3-carbonylbicyclo[2.2.1]heptane.

9. Process according to claims 1 and 2 for the preparation of the compound HomoPyroGlu-($N^{\pi}$-Me)His-ABH-$NH_2$, its diastereoisomers, enantiomers and epimers, and also its addition salts with a pharmaceutically acceptable acid, HomoPyroGlu representing the radical 2-piperidinone-6-carbonyl, ($N^{\pi}$-Me)His the radical 3-methylhistidyl and ABH the radical 2-aza-3-carbonylbicyclo[2.2.1]heptane.

10. Process according to claims 1 and 2 for the preparation of the compound HomoPyroGlu-Leu-ABH-$NH_2$, its diastereoisomers, enantiomers and epimers, and also its addition salts with a pharmaceutically acceptable acid, HomoPyroGlu representing the radical 2-piperidinone-6-carbonyl, Leu representing the radical leucyl and ABH the radical 2-aza-3-carbonylbicyclo[2.2.1]heptane.

11. Process according to claims 1 and 2 for the preparation of the compound PyroGlu-His-ABH-$NH_2$, its diastereoisomers, enantiomers and epimers, and also its addition salts with a pharmaceutically acceptable acid, PyroGlu representing the radical 2-pyrrolidone-5-carbonyl, His representing the radical histidyl and ABH the radical 2-aza-3-carbonylbicyclo[2.2.1]heptane.

12. Process according to claims 1 and 2 for the preparation of the compound HomoPyroGlu-His-ABH-$NH_2$, its diastereoisomers, enantiomers and epimers, and also its addition salts with a pharmaceutically acceptable acid, HomoPyroGlu representing the radical 2-piperidinone-6-carbonyl, His the radical histidyl and ABH the radical 2-aza-3-carbonylbicyclo[2.2.1]heptane.

13. Process according to claims 1 and 2 for the preparation of the compound HomoPyroGlu-($N^{\tau}$-Me)His-ABH-$NH_2$, its diastereoisomers, enantiomers and epimers, and also its addition salts with a pharmaceutically acceptable acid, HomoPyroGlu representing the radical 2-piperidinone-6-carbonyl, ($N^{\tau}$-Me)His the radical 1-methylhistidyl and ABH the radical 2-aza-3-carbonylbicyclo[2.2.1]heptane.

14. Process according to claims 1, 3 and 5 for the preparation of the compound HomoPyroGlu-Leu-MIABO-$NH_2$, its diastereoisomers, enantiomers and epimers, and also its addition salts with a pharmaceutically acceptable acid, HomoPyroGlu representing the radical 2-piperidinone-6-carbonyl, Leu representing the radical leucyl and MIABO the radical 4-methyl-1-isopropyl-2-aza-3-carbonylbicyclo[2.2.2]octane.

15. Process according to claims 1, 3 and 4 for the preparation of the compound PyroGlu-($N^{\pi}$-Me)His-dMABO-$NH_2$, its diastereoisomers, enantiomers and epimers, and also its addition salts with a pharmaceutically acceptable acid, PyroGlu representing the radical 2-pyrrolidinone-5-carbonyl, ($N^{\pi}$-Me)His the r$_a$dical 3-methylhistidyl and dMABO the radical 1,4-dimethyl-2-aza-3-carbonylbicyclo-[2.2.2]octane.

16. Process according to claim 1 for the preparation of compounds of formula (II) which can be used in the preparation of compounds of formula (I).

**17.** Process according to claim 1 for the preparation of compounds of formula (III) which can be used in the preparation of compounds of formula (I).

**18.** Process according to claim 1 for the preparation of compounds of formula (IV) which can be used in the preparation of compounds of formula (I).

**19.** Process according to claim 1 for the preparation of compounds of formula (V) which can be used in the preparation of compounds of formula (I).

**20.** Process according to claim 1 for the preparation of compounds of formula (VII) which can be used in the preparation of compounds of formula (I).

**21.** Process according to claim 1 for the preparation of compounds of formula (VIII) which can be used in the preparation of compounds of formula (I).

**Claims for the following Contracting State : ES**

**1.** Process for the preparation of compounds of the general formula (I):

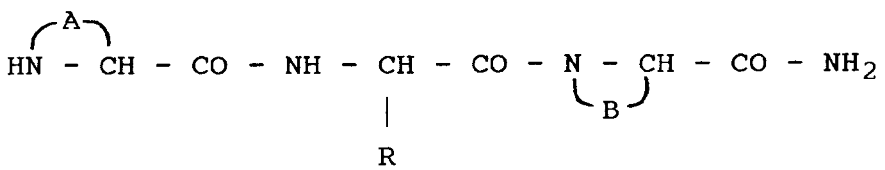

(I)

in which:

A represents, together with the nitrogen and carbon atoms to which it is bonded:
- a 2-oxopyrrolidin-5-yl grouping,
- a 2-oxopiperidin-6-yl grouping,
- a 2,6-dioxo-1,2,3,6-tetrahydropyrimidin-4-yl grouping,
- a 2-oxothiazolidin-4-yl grouping,
- a 2-oxoazetidin-4-yl grouping, or
- a 1-oxo-1,2,3,4-tetrahydroisoquinol-3-yl grouping,

B represents, together with the nitrogen and carbon atoms to which it is bonded, a saturated polycyclic structure selected from 2-azabicyclo-[2.2.1]-heptane and the 1,4-di-(linear and branched ($C_1$-$C_6$)alkyl)-2-azabicyclo[2.2.2]-octanes, and

R represents a hydrogen atom, a linear or branched lower alkyl grouping, or a 4-imidazolylmethyl grouping optionally substituted at one of the nitrogen atoms by a linear or branched lower alkyl radical, the term "lower" indicating that the groupings so described have from 1 to 6 carbon atoms,

their enantiomers, diastereoisomers and epimers and also their addition salts with a pharmaceutically acceptable acid, characterised in that the amine function of an amino acid of formula (II):

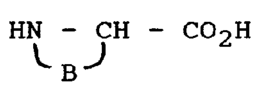

(II)

in which B has the same meaning as in formula (I),
the isomers of which have optionally been separated by a conventional separation technique, is protected by a protecting radical (P), such as tert.-butoxycarbonyl (tBOC) or benzyloxycarbonyl (Z), under the action of a suitable reagent to give a compound of formula (III):

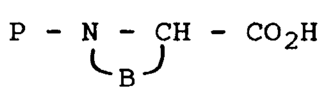

(III)

in which B and P have the same meanings as given above,

on which ethyl chloroformate and then ammonium hydroxide are caused to react, at a temperature of between -15 and 0°C, in the presence of triethylamine,
to give a compound of formula (IV):

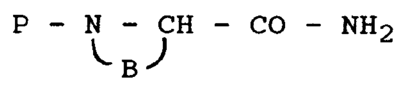 (IV)

in which B and P have the same meanings as given above, which is deprotected by a suitable process, such as, for example, by the action of gaseous HCl in an anhydrous solvent, such as dioxane or ethyl acetate, when P = tBOC or by catalytic hydrogenation when P = Z,
to give a compound of formula (V):

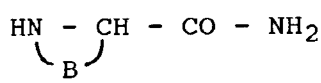 (V)

in which B has the same meaning as in formula (I),
the isomers of which are, if desired, separated by a conventional separation technique,
which is coupled with a second protected amino acid of formula (VI):

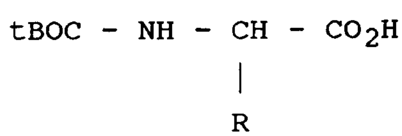 (VI)

in which R has the same meaning as in formula (I),
to give a compound of formula (VII):

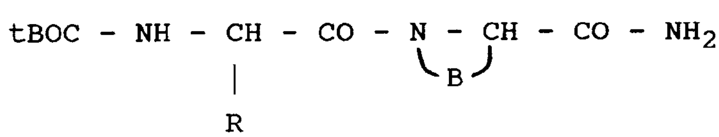 (VII)

in which R and B have the same meanings as in formula (I), the diastereoisomers or enantiomers of which are, if desired, separated by a conventional separation technique,
which is then deprotected by the action of gaseous HCl in an anhydrous solvent, such as, for example, dioxane or ethyl acetate, to give a compound of formula (VIII):

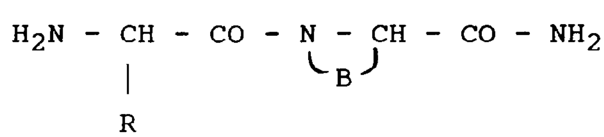 (VIII)

in which R and B have the same meanings as in formula (I),
which is coupled with a third optionally protected amino acid of formula (IX):

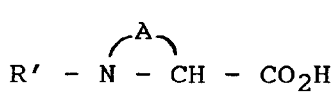 (IX)

in which R' is a hydrogen atom, or a protecting grouping, such as, for example, benzyloxycarbonyl (Z), or with an activated ester of that optionally protected amino acid, to give:

- either a compound of formula (I) when R' is a hydrogen atom, which is, if desired, converted into its addition salt with a pharmaceutically acceptable acid,

- or a compound of formula (X):

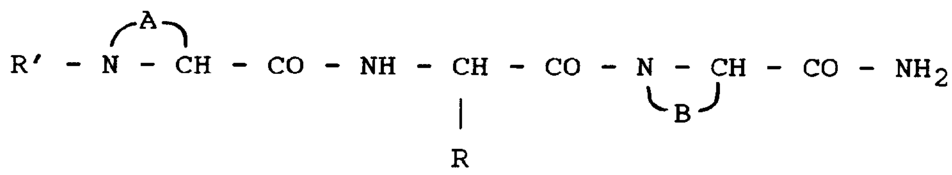 (X)

when R' is a protecting grouping, R and B having the same meanings as in formula (I), which is deprotected by a deprotection technique, such as, for example, catalytic hydrogenation, to give a compound of formula (I),

which is, if desired, converted into its addition salt with a pharmaceutically acceptable acid or separated into its isomers according to a conventional separation technique, which isomers are, if desired, converted into their salts by means of a pharmaceutically acceptable acid.

2. Process according to claim 1 for the preparation of compounds such that B forms, together with the nitrogen and carbon atoms to which it is bonded, a 2-azabicyclo-[2.2.1]heptane ring, their enantiomers, diastereoisomers and epimers and also their addition salts with a pharmaceutically acceptable acid.

3. Process according to claim 1 for the preparation of compounds such that B forms, together with the nitrogen and carbon atoms to which it is bonded, a 1,4-dialkyl-2-azabicyclo[2.2.2]octane ring, their enantiomers, diastereoisomers and epimers and also their addition salts with a pharmaceutically acceptable acid.

4. Process according to claims 1 and 3 for the preparation of compounds such that B forms, together with the nitrogen and carbon atoms to which it is bonded, a 1,4-dimethyl-2-azabicyclo[2.2.2]octane ring, their enantiomers, diastereoisomers and epimers and also their addition salts with a pharmaceutically acceptable acid.

5. Process according to claims 1 and 3 for the preparation of compounds such that B forms, together with the nitrogen and carbon atoms to which it is bonded, a 4-methyl-1-isopropyl-2-azabicyclo[2.2.2]octane ring, their enantiomers, diastereoisomers and epimers and also their addition salts with a pharmaceutically acceptable acid.

6. Process according to claims 1 and 2 for the preparation of the compound PyroGlu-($N^{\tau}$-Me)His-ABH-$NH_2$, its enantiomers, diastereoisomers and epimers, and also its addition salts with a pharmaceutically acceptable acid, PyroGlu representing the radical 2-pyrrolidone-5-carbonyl, ($N^{\tau}$-Me)His representing the radical 1-methylhistidyl and ABH the radical 2-aza-3-carbonylbicyclo-[2.2.1]heptane.

7. Process according to claims 1 and 2 for the preparation of the compound PyroGlu-Leu-ABH-$NH_2$, its diastereoisomers, enantiomers and epimers, and also its addition salts with a pharmaceutically acceptable acid, PyroGlu representing the radical 2-pyrrolidone-5-carbonyl, Leu representing the radical leucyl and ABH the radical 2-aza-3-carbonylbicyclo[2.2.1]heptane.

8. Process according to claims 1 and 2 for the preparation of the compound PyroGlu-($N^{\pi}$-Me)His-ABH-$NH_2$, its diastereoisomers, enantiomers and epimers, and also its addition salts with a pharmaceutically acceptable acid, PyroGlu representing the radical 2-pyrrolidone-5-carbonyl, ($N^{\pi}$-Me)His the radical 3-methylhistidyl and ABH the radical 2-aza-3-carbonylbicyclo[2.2.1]heptane.

9. Process according to claims 1 and 2 for the preparation of the compound HomoPyroGlu-($N^{\pi}$-Me)His-ABH-$NH_2$, its diastereoisomers, enantiomers and epimers, and also its addition salts with a pharmaceutically acceptable acid, HomoPyroGlu representing the radical 2-piperidinone-6-carbonyl, ($N^{\pi}$-Me)His the radical 3-methylhistidyl and ABH the radical 2-aza-3-carbonylbicyclo[2.2.1]heptane.

10. Process according to claims 1 and 2 for the preparation of the compound HomoPyroGlu-Leu-ABH-$NH_2$, its diastereoisomers, enantiomers and epimers, and also its addition salts with a pharmaceutically acceptable acid, HomoPyroGlu representing the radical 2-piperidinone-6-carbonyl, Leu representing the radical

leucyl and ABH the radical 2-aza-3-carbonylbicyclo[2.2.1]heptane.

**11.** Process according to claims 1 and 2 for the preparation of the compound PyroGlu-His-ABH-$NH_2$, its diastereoisomers, enantiomers and epimers, and also its addition salts with a pharmaceutically acceptable acid, PyroGlu representing the radical 2-pyrrolidone-5-carbonyl, His representing the radical histidyl and ABH the radical 2-aza-3-carbonylbicyclo[2.2.1]heptane.

**12.** Process according to claims 1 and 2 for the preparation of the compound HomoPyroGlu-His-ABH-$NH_2$, its diastereoisomers, enantiomers and epimers, and also its addition salts with a pharmaceutically acceptable acid, HomoPyroGlu representing the radical 2-piperidinone-6-carbonyl, His the radical histidyl and ABH the radical 2-aza-3-carbonylbicyclo[2.2.1]heptane.

**13.** Process according to claims 1 and 2 for the preparation of the compound HomoPyroGlu-($N^{\tau}$-Me)His-ABH-$NH_2$, its diastereoisomers, enantiomers and epimers, and also its addition salts with a pharmaceutically acceptable acid, HomoPyroGlu representing the radical 2-piperidinone-6-carbonyl, ($N^{\tau}$-Me)His the radical 1-methylhistidyl and ABH the radical 2-aza-3-carbonylbicyclo[2.2.1]heptane.

**14.** Process according to claims 1, 3 and 5 for the preparation of the compound HomoPyroGlu-Leu-MIABO-$NH_2$, its diastereoisomers, enantiomers and epimers, and also its addition salts with a pharmaceutically acceptable acid, HomoPyroGlu representing the radical 2-piperidinone-6-carbonyl, Leu representing the radical leucyl and MIABO the radical 4-methyl-1-isopropyl-2-aza-3-carbonylbicyclo[2.2.2]octane.

**15.** Process according to claims 1, 3 and 4 for the preparation of the compound PyroGlu-($N^{\pi}$-Me)His-dMABO-$NH_2$, its diastereoisomers, enantiomers and epimers, and also its addition salts with a pharmaceutically acceptable acid, PyroGlu representing the radical 2-pyrrolidinone-5-carbonyl, ($N^{\pi}$-Me)His the radical 3-methylhistidyl and dMABO the radical 1,4-dimethyl-2-aza-3-carbonylbicyclo-[2.2.2]octane.

**16.** Process according to claim 1 for the preparation of compounds of formula (II) which can be used in the preparation of compounds of formula (I).

**17.** Process according to claim 1 for the preparation of compounds of formula (III) which can be used in the preparation of compounds of formula (I).

**18.** Process according to claim 1 for the preparation of compounds of formula (IV) which can be used in the preparation of compounds of formula (I).

**19.** Process according to claim 1 for the preparation of compounds of formula (V) which can be used in the preparation of compounds of formula (I).

**20.** Process according to claim 1 for the preparation of compounds of formula (VII) which can be used in the preparation of compounds of formula (I).

**21.** Process according to claim 1 for the preparation of compounds of formula (VIII) which can be used in the preparation of compounds of formula (I).